# EUROPEAN PATENT SPECIFICATION

(11) **EP 0 464 155 B1**
(45) Date of publication and mention of the grant of the patent: **07.05.1997**
(21) Application number: 90907823.0
(22) Date of filing: 22.03.1990
(51) Int. Cl.: A61K 38/27, A61K 38/16, C07K 1/00

(54) **ENDOTHELIAL CELL GROWTH FACTOR, ISOLATION AND EXPRESSION**
ENDOTHELIALER ZELLWACHSFAKTOR, ISOLIERUNG UND EXPRESSION
FACTEUR DE CROISSANCE CELLULAIRE ENDOTHELIALE, SON ISOLATION ET SON EXPRESSION

(30) Priority: 24.03.1989 US 328181; 02.05.1989 US 346165; 01.06.1989 US 360235
(43) Date of publication of application: 08.01.1992
(73) Proprietor: THE REGENTS OF THE UNIVERSITY OF CALIFORNIA, Oakland,California 94612-3550 (US)
(72) Inventor: FERRARA, Napoleone, Belvedere, CA 94920 (US); GOSPODAROWICZ, Denis, San Francisco, CA 94127 (US); PLOUET, Jean, San Francisco, CA 94116 (US)
(74) Representative: Harrison, David Christopher
(86) International application number: US9001568
(87) International publication number: WO9011084

(56) References cited:
- EP-A- 0 370 989
- EP-A- 0 399 816
- WO-A-84/02135
- WO-A-90/13649
- WO-A-91/02058
- US-A- 5 008 196
- MONOKINES AND OTHER NON-LYMPHOCYTIC CYTOKINES, 1988, pages 325-328, Alan R. Liss, Inc., New York, US; G. BECK et al.: "Isolation and characterization of a vascular permeability factor from stimulated U937 cells"
- PROCEEDINGS OF THE NATIONAL ACADEMY OF SCIENCES USA, vol. 86, October 1989, pages 7311-7315, Washington, US; D. GOSPODAROWICZ et al.: "Isolation and characterization of a vascular endothelial cell mitogen produced by pituitary- derived folliculo stellate cells"
- SCIENCE, vol. 246, 8th December 1989, pages 1306-1309, Lancaster, PA, US; D.W. LEUNG et al.: "Vascular endothelial growth factor is a secreted angiogenic mitogen"
- SCIENCE, vol. 246, 8th December 1989, pages 1309-1312, Lancaster, PA, US; P.J. KECK et al.: "Vascular permeability factor, an endothelial cell mitogen related to PDGF"
- CANCER RESEARCH, vol. 46, November 1986, pages 5629-5632, Philadelpha, US; D.R. SENGER et at.: "A highly conserved vascular permeability factor secreted by a variety of human and rodent cell lines"
- BURGESS et al.: "Multiple Forms of Endothelial Cell Growth Factor", J. Biol. Chem. Vol. 260, No. (21) p. 11389-11392 (1985). See Abstract and results section
- WINKLES et al.: "Human Vascular Smooth Muscle Cells both express and respond to heparin-binding growth factor I (endothelial cell growth factor)", Proc. Natl. Acad. Sci. USA 84, p. 7124-7128, (October 1987). See entire document

## Description

### BACKGROUND OF INVENTION

The present invention relates to a novel growth factor for vascular endothelial cells identified in media condition of cultured bovine pituitary follicular cells and of murine tumor cells. The invention also relates to isolation, purification and cloning of the growth factor.

### Description of the Problem and Related Art

Numerical references in parenthesis in the text refer to the publications listed below in the Reference Section.

Angiogenesis is a multi-step phenomenon which involves capillary endothelial cell proliferation, migration and tissue infiltration (1). It plays a central role in a variety of physiological and pathological processes such as embryonic development, wound healing, atherosclerosis and tumor growth (1,2). Several factors induce angiogenesis have recently been isolated and characterized. Among these are the acidic and basic form of fibroblast growth factor (FGF), both capable of stimulating capillary endothelial cell growth in vitro as well as being chemotactic for that cell type (2). In addition, both acidic and basic FGF stimulate collagenase activity and plasminogen activator production while blocking the activity of plasminogen inhibitor (3,4). These enzymes are involved in the breakdown of the capillary basement membrane, an event required in order for angiogenesis to take place (1). Other growth factors such as tumor necrosis factor alpha (TNFa), transforming growth factor beta (TGFβ), transforming growth factor alpha (TGFa), and epidermal growth factor (EGF) are also angiogenic in vivo (5-8). However, with the exception of TGFa and EGF at high concentrations (7), these growth factors are not mitogenic for capillary endothelial cells (5,6); their action on the angiogenic process is therefore probably indirect, resulting from such activities as the attraction of macrophages by chemotaxis (9,10) which in turn release direct angiogenic factor(s), one of which could be basic FGF (11).

A number of growth factors, such as acidic and basic FGF, PDGF and EGF, are broadly mitogenic for a number of cell types. This broad mitogenicity is desirable in many types of wound healing applications. There are, however, specific types of wound healing applications in which it would be more desirable to employ growth factors having more cell-specific mitogenic activity. For example, following vascular graft surgery or balloon angioplasty, it would be highly desirable to employ a wound healing agent incorporating mitogenic factor having mitogenic activity that is highly specific for vascular endothelial cells. At present, no highly suitable mitogenic factor exists for this type of application.

In the course of our studies on the localization of basic FGF in various tissues, it was observed that, in the pituitary gland, folliculo stellate cells are the main producers of bFGF (18). Although the medium conditioned by those cells was found to be strongly mitogenic for capillary endothelial cells, little if any bFGF is present in it, thus suggesting that, in addition to synthesizing bFGF, these cells are also capable of producing another endothelial cell mitogen. To date, however, this mitogenic activity has not been purified or characterized.

Art of Interest--The following publications are of interest as background in this art.
1. Folkman, J. (1986) Cancer Res. 46, 467-473.
2. Gospodarowicz, D., et al. (1987) Endocrine Reviews 8, 95-114.
3. Saksela, O., et al. (1987) J. Cell Biol. 107, 957-962.
4. Montesano, R., et al. (1986) Proc. Natl. Acad. Sci. U.S.A. 83, 7297-7301.
5. Frater-Schroeder, M. (1987) Proc. Natl. Acad. Sci. U.S.A. 84,5277-5281.
6. Sporn, M.B., et al. (1986) Proc. Natl. Acad. Sci. U.S.A. 83,4167-4272.
7. Schreiber, A.B., et al. (1986) Science 232, 1250-1253.
8. Gospodarowicz, D., et al. (1979) Expt. Eve Res. 28, 501-514.
9. Leibovich, S., et al. (1987) Nature 329, 630-632.
10. Wahl, S.M., et al. (1987) Proc. Natl. Acad. Sci. U.S.A. 84,5788-5792.
11. Baird, A., et al. (1985) Biochem. Biophys. Res. Commun. 126, 358-363.
12. Abraham, J.A., et al. (1986) Science, 233, 545-547.
13. Jaye, M., et al. (1985) Science 233, 541-545.
14. Schweigerer, L., et al. (1987) Nature, 325, 257-259.
15. Moscatelli, D., et al. (1986) J. Cell. Physiol. 129, 273-276.
16. Vlodavsky, I., et al. (1987) Proc. Natl. Acad. Sci. U.S.A. 84,2292-2296.
17. Ferrara, N., et al. (1987) Proc. Natl. Acad. Sci. U.S.A. 84,5773-5777.
18. Gospodarowicz, D., et al. (1984) Proc Natl. Acad. Sci. U.S.A. 81,6963,6967.
19. Gospodarowicz, D., et al. (1988) Europ. J. Cell. Biol., 46,144-151.
20. Ferrara, N., et al. (1986) Methods Enzymol. 124, 235-253.
21. Gospodarowicz, D., et al. (1983) J. Cell. Biol. 7, 1677-1685.
22. Gospodarowicz, D., et al. (1986) J. Cell. Physiol., 127, 121-136.
23. Gospodarowicz, D., et al. (1977) Endocrinology 100, 1108-1120.
24. Gospodarowicz, D., et al. (1977) Endocrinology 100, 1080-1089.
25. Gospodarowicz, D., et al. (1977) Ext. Eye. Res. 25, 75-89.
26. Neufeld, G., et al. (1986) Regulatory Peptides 13, 293-305.
27. Weissman, B.E., et al. (1983) Cell 32, 599-606.
28. McConahey, P., et al. (1966) Int. Arch. Allergy 29, 185-189.
29. Laemmli, U.K. (1970) Nature, (London) 227, 680-685.
30. Klagsburn, M., et al. Proc. Natl. Sci. U.S.A. 82, 805-809.
31. Gospodarowicz, D., (1987) Methods Enzymol. 147, 106-119.
32. Kudlow, J.E., et al. (1988) In Biology of growth factor. Adv. in Exptl. Med. and Biol. Pelnum Press New York 234,
105-126.
33. Frater-Schroder, M., et al. (1986) Biochem. Biophys. Res. Commun. 137, 295-302.
34. Baird, A., et al. (1986) Biochem. Biophys. Res. Commun. 138, 476-482.
35. Lipman, D.G., et al. (1985) Science (Wash., D.C.) 227, 1435-1441.
36. Rubin, J.S., et al. (1989) Proc. Natl. Acad. Sci. U.S.A. 86, 803-806.
37. Miyazono, K., et al. (1987) J. Biol. Chem., 262,4098-4103.
38. Farquhar, M.G., et al. In The Anterior Pituitary Gland (Tixier Vidal A., and Farquhar M.G. eds.) Acad. Press, New York 1975, pp 82-102.
39. Gon, G. Shirasawa, et al. (1987) Anat. Rec. 217, 371-384.
40. Carpenter, G., et al. (1985) Anal. Biochem. 153, 279-282.
41. Ferrara, N., et al. (1987) Am. J. Physiol. 252, E304-312.
42. Ferrara, N., et al. (1988) Biochem. Bioph^{y}s. Res. Comm., 157, 1376-1382.
43. Abraham, J., et al. (1986) EMBOJ.. 5, 2523-2529.
44. Water, P., et al. (1981) J. Cell Biol., 91, 557-561.
45. Klagsburn, M., et al. (1986) Proc. Natl. Acad Sci. USA, 83, 2448-2452.
46. Neufeld, G., et al. (1987) Endocrinology. 121, 597-602.
47. Schweigerer, L., et al. (1988) Exp. Eye Res., 46(1), 71-80.
48. Schweigerer, L., et al. (1987) Endocrinology 120, 796-802.
49. Shing, Y, et al. (1984), Science 223, 1296-1299.
50. Jaffe, E.A., et al. (1972) J. Clin. Inv. 51, 46a.
51. Folkman, J. (1982) In: Pathobiology of the Endothelial Cell. (Edited by Nossel, H.L., and Vogel, H.J.) pp 79-93,
Academic Press, New York.
52. D'Amore, PA., et al. (1981) Proc. Natl. Acad. Sci. U.S.A, 78,3068-3072.
53. Pheel, D.M., et al. (1985) In Vitro, 16, 526-538.
54. Henzel, W.J., et al. (1987) J. Chromatograph., 404,41-52.
55. Morrissey, J.H. (1981) Anal. Biochem., 117, 307-310.
56. Baird, A., et al. (1986) Recent Prog. Hormone Res., 42, 143-186.
57. Roberts, R., et al. (1988) Nature, 332, 376-378.
58. Maciag, T, et al. (1984) Science, 225, 932-935.
59. Lobb, R.R., et al. (1984) Biochemistry. 23,6295-6299.
60. Folkman, J., et al. (1987), Science, 235, 442-447.
61. Goustin, A.S., et al. (1986) Cancer Res., 46, 1015-1029.
62. Bassett, D.L. (1943) Am. J. Anat., 73,251-259.
63. Gospodarowicz, D., et al. U.S. Patent No. 4,785,079, issued November 15, 1988.
64. B. Gumbiner, et al. (1981) Proc. Natl. Acad. U.S.A. 78, 318.
65. S. Blam, et al. (1988) Oncogene 3, 129.

All of the references and/or patents cited in this application are incorporated herein by reference.

These references appear in the text in parentheses (1).

Previously published research describes the culture of homogeneous populations of bovine pituitary follicular or folliculo-stellate cells (FC) (20) and subsequently characterized them as ion transport elements, possibly involved in the regulation of ion composition and osmolarity of the interstitial fluid in the adenohypophysial cell cords (41, 42). It is also reported that FC produce the angiogenic mitogen basic fibroblast growth factor (bFGF) (17).

The gene for bFGF (43), similarly to the gene for acidic fibroblast growth factor (aFGF) (13), does not code for a conventional signal peptide, required for the extracellular transport of proteins according to classic secretory pathways (44). Accordingly, the growth factor is not appreciably secreted in the medium (15,45) and responsive cell types are dependent on exogenous bFGF for optimal proliferation in culture, even though they may contain significant intracellular concentrations of mitogen (46,47,48).

It was initially observed, however, that the medium conditioned by bovine pituitary FC is mitogenic for adrenal-cortex-derived capillary endothelial cells. Interestingly, these cells are responsive either to bFGF or aFGF but are not stimulated to proliferate by EGF, TGF alfa, TGF beta, PDGF, insulin or TNF (2). These observations led us to consider the possibility that an endothelial cell growth factor distinct from FGF and possibly any other known growth factor may be secreted by cultured FC.

The present invention describes the purification and biological characterizations of such a novel growth factor. Its unique N-terminal amino acid sequence, as well as its specificity for vascular endothelial cells, distinguishes it from any previously described growth factor.

### SUMMARY OF THE INVENTION

In accordance with the present invention, there is provided a novel growth factor in isolated form, that is, unaccompanied by impurities which normally accompany the native molecule when it is produced in vivo. The growth factor of the invention shall be referred to herein as "folliculo stellate-derived endothelial cell growth factor" (FSdGF), since it was originally isolated from bovine folliculo stellate cells, or as "vascular endothelial growth factor" (VEGF). It is to be understood, however, that these terms are intended herein to encompass the protein, regardless of its source or manner of production. For example, the provision herein of FSdGF in isolated form provides the means for isolating cloned DNA sequences encoding the protein, so that it can be produced in commercial quantities using the known techniques of recombinant DNA technology. Furthermore, FSdGF herein is intended to encompass the corresponding proteins produced by other than bovine species, e.g. the human protein, even though it is known that minor variations in amino acid sequence from species to species may occur which do not significantly affect the useful activities of a protein. Using materials and procedures described herein those skilled in the art can obtain, for example, the corresponding human protein by isolating and expressing cloned DNA sequences encoding the protein. Also included within the scope of the term "FSdGF" herein are biologically active fragments thereof, as well as N-terminally and/or C-terminally extended versions thereof, which retain qualitatively the biological activities of the FSdGF described herein. While the form of FSdGF which was isolated using procedures described herein is apparently glycosylated, it is known that production of proteins by recombinant means in certain procaryotic hosts such as E. coli generally does not result in glycosylated forms of the protein, but that the resulting unglycosylated forms are often quite useful. Accordingly, the term "FSdGF" encompasses glycosylate and unglycosylated forms of the molecule, provided that they retain qualitatively the biological activities described herein.

FSdGF is a dimeric protein of approximately 43-45 kd, as determined by SDS polyacrylamide gel electrophoresis under non-reducing conditions. It appears to exhibit cell-specific mitogenic activity on vascular endothelial cells. Consequently, FSdGF will find use as a growth factor in a variety of wound healing applications in which it is desired to promote re-endothelialization in the vascular system. FSdGF will be particularly useful as a post-operative wound healing agent in both vascular graft surgery and balloon angioplasty FSdGF can also be employed as a mitogenic agent for growing endothelial cells in vitro. Yet another application for FSdGF is the promotion of vascular wound healing following myocardial infarction.

In accordance with the invention, FSdGF can be obtained in isolated form from conditioned cell culture media containing FSdGF by a process which includes the steps of ammonium sulfate precipitation; heparin sepharose affinity chromatography; exclusion gel chromatography; cation exchange chromatography; and optionally reverse phase high pressure liquid chromatography

### BRIEF DESCRIPTION OF THE FIGURES

Figure 1A shows the heparin sepharose affinity chromatography (HSAC) of the condition medium by bovine FS cells.
Figure 1 B shows the gel exclusion chromatography of the partially purified HSAC fractions on BioGel P-60.
Figure 1 C shows the chromatographic results on Mono S of the bioactive BioGell P-60 fraction.
Figure 2A shows the reverse phase HPLC of the Mono S purified and bioactive fractions, and a comparison of the ability of the FS cell conditioned medium at various stages of purification to stimulate the proliferation of low density ACE cells cultures.
Figure 2B is a comparison of the ability of FS cell conditioned medium at various stages of purification to stimulate the proliferation of low density ACE cell cultures.
Figure 3 is the NaDodS0₄"PAGE of the bioactive fraction purified by RP C₄ HPLC.
Figure 4A, 4B and 4C are a comparison between the ability of bovine pituitary derived bFGF versus FSdGF to stimulate growth of HUE cells (A), ACE cells (B) and BHK-21 cells (C).
Figure 5 is a comparison of the ability of bFGF versus FSdGF to stimulate the proliferation of BCE cells, granulosa cells, adrenal cortex cells and BALB/MK cells.
Figure 6 is a spectrum of reduced/alkylated/lysine blocked FEGF.
Figure 7 is a trace of the trypsin digestion of the novel growth factor.
Figure 8 is a graph of the reversed-phase high performance liquid chromatography of bovine follicular cells mitogen activity.
Figure 9A and 9B are a NadoS04/PAGE (12.5 acrylamide) analysis of the most bioactive fraction from the previously shown HPLC profile.
Figure 10 shows a graph of the proliferation of the low density adrenal cortex derived endothelial cells as a function of time.
Figure 11 is a dose-response growth curve of adrenal cortex derived capillary endothelial cells in the presence of purified VEGF.
Figure 12 is a graph of the effects of VEGF on different cell types.
Figure 13A shows the heparin sepharose affinity chromatography (HSAC) of the condition medium by FS cells.
Figure 13B shows the gel exclusion chromatography of the partially purified HSAC fractions on Sepharose G/100.
Figure 13C shows the chromatographic results of Mono S of the bioactive BioGel P-60 fraction.
Figure 13D shows the reverse phase HPLC of the Mono S purified and bioactive fractions, and a comparison of the ability of the FS cell condition medium at various states of purification to stimulate the proliferation of low density ACE cell cultures.
Figure 14 is the NaDoDSO₄/PAGE of the bioactive fraction purified by RP C₄ HPLC.
Figures 15A, 15B and 15C are a comparison between the ability of murine derived bFGF versus FSdGF to stimulate growth of HUE cells (A), ACE cells (B) and BHK-21 cells (C).
Figure 16 is a comparison of the ability of bFGF versus murine growth factor to stimulate the proliferation of BCE cells, granulosa cells, adrenal cortex cells and BALB/MK cells.

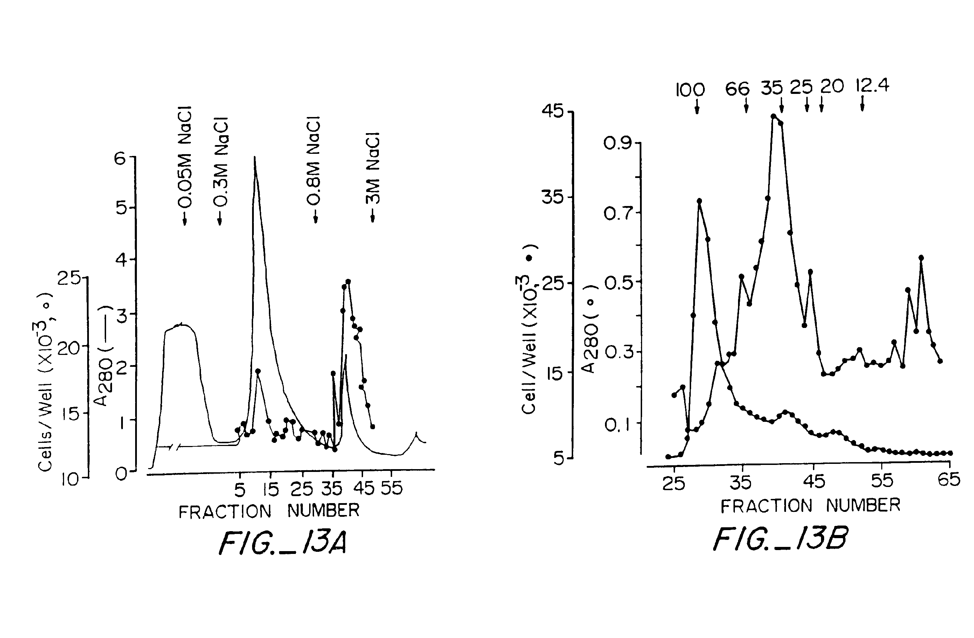

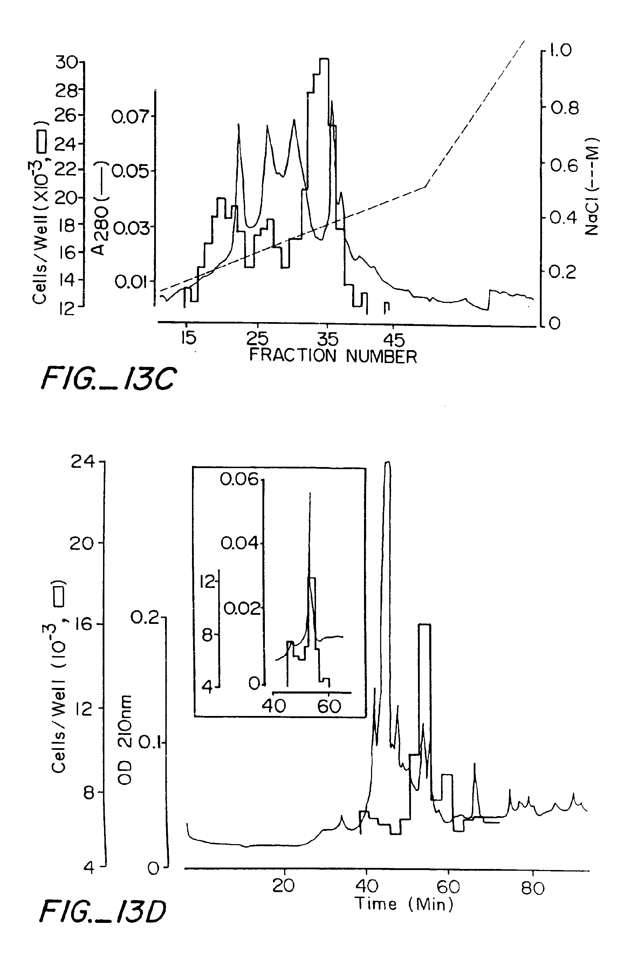

Figures 13 to 16 refer to the murine source (AtT20) for the growth factor.

### Detailed Description of the Invention

### Definitions

As used herein, the following abbreviations have the indicated meanings:
aFGF acidic fibroblast growth factor
bFGF basic fibroblast growth factor
PDGF platelet derived growth factor
TGFa transforming growth factor a
TGFj3 transforming growth factor β
EGF epidermal growth factor
PDECGF platelet derived endothelial cell growth factor
FS folliculo stellate cells
FSdGF folliculo stellate derived growth factor
STV 0.01 M sodium phosphate (pH 7.4), 0.9% NaCl, 0.05% trypsin, 0.02% EDTA
CS calf serum
FCS fetal calf serum
PBS phosphate buffered saline
HSAC heparin sepharose affinity chromatography
RP-HPLC reverse phase high pressure liquid chromatography
FPLC fast high pressure liquid chromatography
ACE cells adrenal cortex-derived capillary endothelial cells
HUE cells human umbilical endothelial cells
BCE cells bovine corneal endothelial cells
RIA radioimmunoassay
BSA bovine serum albumin
BHK21 baby hamster kidney-derived fibroblast clone 21
Na Dod S0₄ sodium dodecyl sulfate
PAGE polyacrylamide gel electrophoresis
MW molecular weight
kDa kilo Dalton
DMEM Dublbecco's modified Eagle's medium

### Materials

Bio Gel P-60 (Registered Trade Mark), Bio Rad protein assay kit, silver nitrate stain kit and low molecular weight standards for Na Dod S0₄/PAGE were from Bio Rad (Richmond, CA). Heparin Sepharose (Registered Trade Mark), Concanavalin A Sepharose, and Mono S (Registered Trade Mark) column HR5/5 were obtained from Pharmacia (Piscataway, NJ). The Vydac C₄ reverse phase column was purchased from Separation Group (Hesperia, CA). Dulbecco's Modified Eagle's Medium (DMEM) was obtained from Grand Island Biological Co. (Grand Island, NY). STV (saline containing 0.05% Trypsin, 0.01 M sodium phosphate pH 7.3 and 0.02% EDTA) was obtained from Difco Lab (Detroit, MI). Calf serum (CS) and fetal calf serum (FCS) were obtained by HyClone Sterile Systems, Inc. (Logan, UT). Tissue culture dishes were purchased from Falcon Plastics (Oxnard, CA), except for large scale Nunc culture plates (600 cm²) which were from Applied Scientific (San Francisco, CA). Gentamicin was obtained from Schering Co. (Kenilworth, NJ), and Fungizone was purchased from E.R. Squibb and Sons (Princeton, NJ). Leupeptin, gelatin, transferring and insulin were Sigma (St. Louis, MO). Pituitary derived basic FGF and neutralizing rabbit polyclonal antibodies directed against basic FGF were prepared as previously described (18,19).

### Cell culture

Pituitary derived folliculo stellate (FS) cell cultures were prepared and characterized as previously described (17,20). Confluent cultures, which consisted of homogeneous dome-forming cell monolayers, were dissociated by exposure to STV supplemented with Na₂EDTA to a final concentration of 0.3% (4-5 min, 24°C). The cells were then seeded at a split ratio of 1:10 into large-scale culture plates and grown in the presence of DMEM supplemented with 5% CS, 5% FSC, 50 µg/ml gentamicin and 2.5 µg/ml Fungizone (18). Upon reach confluency, cultures were further passaged or exposed to serum free medium (see below). Cultures of human umbilical endothelial cells (21), bovine brain and adrenal cortex derived capillary endothelial cells (22), bovine granulosa cells (23), adrenal cortex cells (24), corneal endothelial cells (25), baby hamster kidney cells clone 21 (BHK-21)(26). and BALB/MK mouse epidermal keratinocytes (27) (a gift from NIH NCI, Bethesda, MD) were maintained as previously described (21-27).

### Preparation of conditioned medium

Early passage FS cells were plated onto 600 cm² Nunc plates and grown to confluence over 4 to 5 days in DMEM supplemented with 5% CS, 5% FCS and antibodies as described above. Once dome formation was observed, the monolayers were washed twice with 25 ml of phosphate buffered saline prior to the addition of 150 ml per plate of DMEM supplemented with 50 µg/ml gentamicin, 25 µg/ml Fungizone, 10 µg/ml leupeptin, 5 µg/ml insulin and 10 µg/ml transferrin. After 48 or 72 hr, culture fluids were collected and replaced with the same amount of fresh serum free medium. Collections could be made for a month or more without visible deterioration of the monolayer.

### Isolation procedure

Conditioned medium collected from the confluent monolayers was centrifuged (10,000g, 15 min) in order to remove floating cells and cell debris. The pH of the supernatant was then adjusted to 5.6 with 6N HCI. Ammonium sulfate (NH₄)₂SO₄ (520 g/liter) was added, and the suspension was set for 6 hr 4°C, the precipitate was then collected by centrifugation (10,000g, 30 min), redissolved in PBS, and stored at -70°C.

For final isolation, the precipitates from 3 collections (21 liters total of conditioned medium, starting material) were thawed, pooled and then dialyzed overnight at 4°C against 10m mM Tris-HCI pH 7.3, 50 mM NaCl. Following dialysis the insoluble material was removed by centrifugation (10,000 g, 30 min) and the supernatant was loaded onto a heparin Sepharose resin (20ml) that had been equilibrated in 10 mM Tris-HCI pH 7.3, 50 mM NaCl. The resin was washed extensively with the equilibration buffer until the absorbance had returned to baseline, and was then eluted stepwise with increasing NaCI concentrations (0.15 M, 0.45 M, 1 M and 3M NaCI). Aliquots were removed from the fractions for cell proliferation assays, and fractions with the highest bioactivity were pooled and concentrated to 1 ml with an Amicon ultrafiltration cell (Model 12) equipped with a Diaflo YM 10 ultrafiltration membrane.

The concentrated sample was loaded onto a Bio Gel P-60 column (100-200 mesh 1 x 95 cm) equilibrated at 4°C in PBS and was eluted with PBS. The Bio Gel P-60 column may be replaced with a Sephadex G-100 which appears to be more efficient. Aliquots of each fraction were taken for cell proliferation assay and the bioactive fractions were pooled, and diluted two fold with 20 mM HEPES pH 8.3. The sample was then applied with a Super loop onto a Mono S column linked to a FPLC system (Pharmacia, Piscataway, NJ). Elution was achieved with a multilineal gradient (20 mM HEPES pH 8.3 to 20 mM HEPES pH 8.3, 1 M NaCI). After fraction aliquots were tested for bioactivity, the active fractions were pooled and loaded onto a Vydac C₄ HPLC column that had been equilibrated in 0.1% trifluoroacetic acid (TFA), 20% acetonitrile. The column was eluted with a linear gradient of 20 to 45% aqueous acetonitrile. Aliquots for the bioassay were then taken, and the column fractions were stored frozen at -70°C.

### Cell proliferation assays

The mitogenic activity of the column fractions and purified samples was determined by using as target cells adrenal cortex-derived capillary endothelial cells (ACE cells) (22). Stock cultures, maintained in the presence of the DMEM supplemented with 10% CS, 50 µm/ml gentamicin, and 0.25 pm/ml Fungizone were passaged weekly on gelatinized tissue culture dishes at a split ratio of 1:10.

For mitogenic assay, cells were seeded in 12 well cluster plates at a density of 5 x 10³ cells per well in 1 ml DMEM supplemented with 10% calf serum and antibiotics, as described previously (19). Six hours later, a set of triplicate wells was trypsinized, and cells were counted to determine the plating efficiency. Ten microliter aliquots of the appropriate dilution of each sample, as indicated in the figure legend detailed descriptions below, were then added in triplicate to wells in the dishes on days 0 and 2. After 4 days in culture, the plates were trysinized, and cell densities were determined with a Coulter counter (Coulter Electronics, Hialeah, FL).

The mitogenic activity of the final purified material was also tested on human umbilical endothelial cells, bovine granulosa cells, adrenal cortex cells, corneal endothelial cells, BHK-21 cells and BALB/MK mouse epidermal keratinocytes. For assaying, cells were seeded at an initial density of 2 or 4 x 10⁴ cells/35-mm dish. Assays were conducted as described for bovine vascular endothelial cells.

### Na Dod S04/PAGE

Samples were reacted with 250 µCi of Na¹²⁵l using the chloramine T method of iodination (28). After TCA precipitation in the presence of ovalbumin carrier (100 µg/ml), the ¹²⁵l-labelled samples (2.5-16 x 10⁴ cpm in 10 µl) were analyzed by Na Dod SO₄/PAGE, (15% polyacrylamide, ref. 29) under reducing or non reducing conditions. After electrophoresis (5 hr, 20 mAmp) the gels were stained with 0.1% Coomassie blue in 50% trichloroacetic acid for 15 min and destained overnight with 7% acetic acid. Gels were then dried and subjected to autoradiography for 6 to 92 hr.

### Protein microsequencing

For protein sequencing, approximately 5 _{w}g (=200 pmol) of protein from the active fractions of the C₄ column were redissolved in 50% trifluoroacetic acid and loaded onto an Applied Biosystems 477A gas-phase protein sequenator. Twelve rounds of Edman degradation were carried out using standard software and chemicals supplied by Applied Biosystems, and identifications of PTH amino acids were made with an automated on-line HPLC column (model 120, Applied Biosystems, Foster City, CA).

### Growth Factor Isolation and Detection

Preliminary experiments indicated that media conditioned by FS cells contained considerable amounts of mitogenic activity for capillary endothelial cells which could not be neutralized by specific aFGF or bFGF neutralizing polyclonal antibodies. Furthermore when applied to a HS affinity column in 0.6 M NaCl, the majority of the activity was not retained. In contrast, aFGF and bFGF are both retained under similar conditions, and elute from HS at NaCI concentrations of 1.1 M and 1.6 M, respectively (18,30,31). Since cultured pituitary cells are known to produce various growth factors (32), the possibility existed that factors, such as TGFa, EGF and/or TGFβ, might also be present in the conditioned medium from FS cells. ACE cells, which do not respond to TGFa, EGF, or PDGF (22) and for which TGFβ is growth inhibitory (33,34), were therefore used to follow the purification of the novel growth factor.

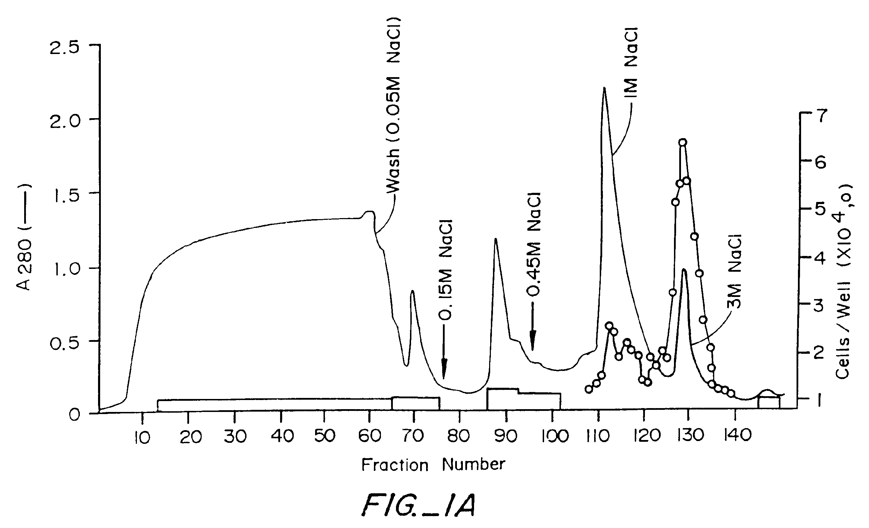

(NH₄)₂SO₄ precipitation provided a convenient way of reducing the volume of the collected conditioned medium from the FS cells to a level suitable for subsequent chromatography HSAC, which has been used for the purification of other growth factors (32, 33) provided an efficient purification step. Material not retained by the column was inactive and accounted for 50% of the total protein loaded (Fig. 1 A). It is likely that the transferrin and insulin components of the cell media were present in the unretained fraction and contributed to the major portion of the proteins. Elution with 0.15 M NaCI yielded a small peak of protein with no bioactivity, while elution with 0.45 M yielded a major peak of protein with 10% of the bioactivity applied to the column. Preliminary experiments had shown that most of the bioactivity could be eluted from HS with 0.6 M NaCl, but that the activity eluted as a broad peak. Therefore, to concentrate the protein peak, elution with 1 M NaCI was carried out. This step gave a relatively narrow protein peak in which 90% of the bioactivity applied on the column was recovered (fig. 1 A). Overall, the HS chromatography resulted in a thirty fold purification, estimated by the protein recovered. Since the growth promoting activity in the starting material was variable, possibly due to the presence of inhibitor, the yield in this step could not be determined exactly (see Table I).

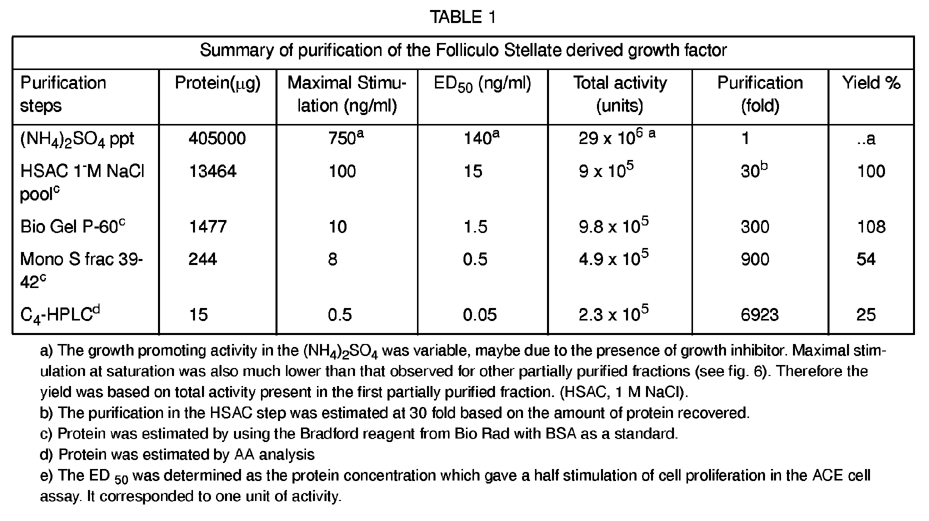

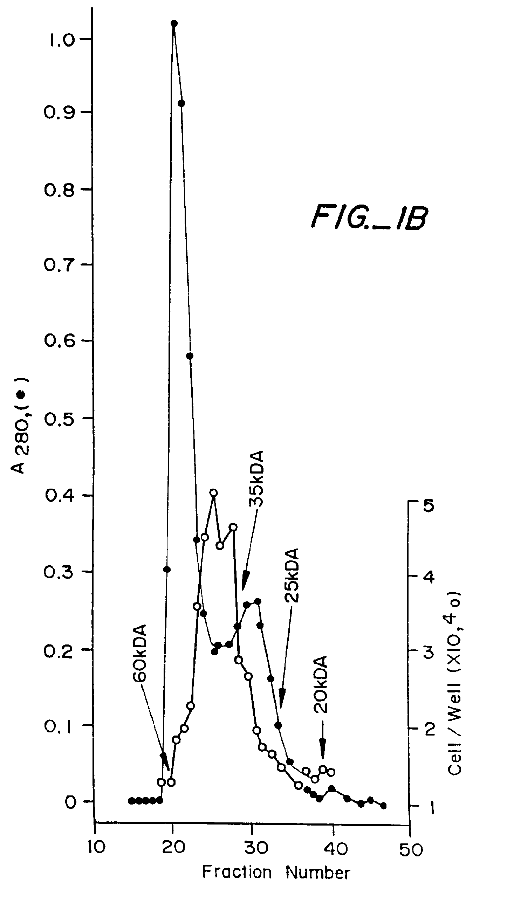

Heparin Sepharose chromatography was followed by gel exclusion chromatography using Bio Gel P-60 (Fig. 1 B). The bioactivity eluted as a major peak with an apparent MW of 40 to 45 kDa. This step resulted in a further ten fold purification with a recovery of 100% (Table 1).

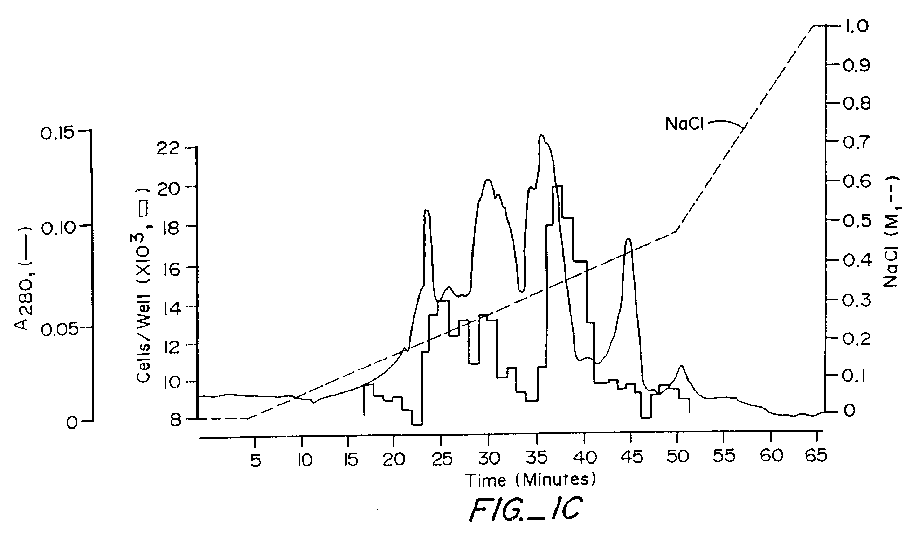

The bioactive fractions from the Bio Gel P-60 column were pooled and applied to a Mono S column (Fig. 1 C). The bioactive profile of the eluted fractions consisted of two minor peaks of bioactivity eluting respectively of 0.23 M NaCI and 0.28 M NaCl, with a major bioactive peak eluting at 0.33 M NaCl. Analysis of the bioactivity present in the various peaks indicated that the 0.33 M NaCI fractions contained five fold more activity than either the 0.23 M or 0.28 M NaCI peak. The Mono S step gave a further three fold increase in specific biological activity over the Bio Gel P-60 step; recovery was about 50% (Table 1). The two other bioactive peaks accounted for the remainder of the bioactivity.

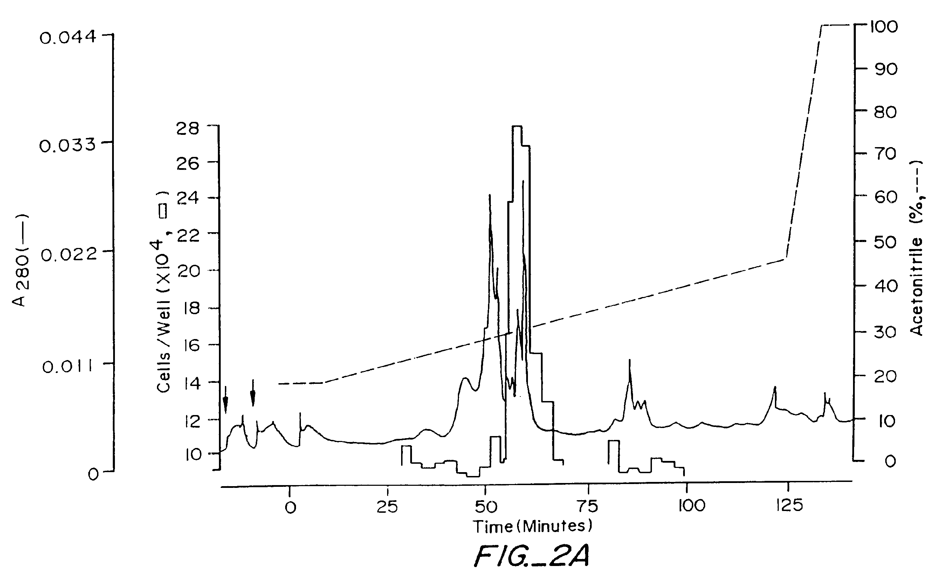

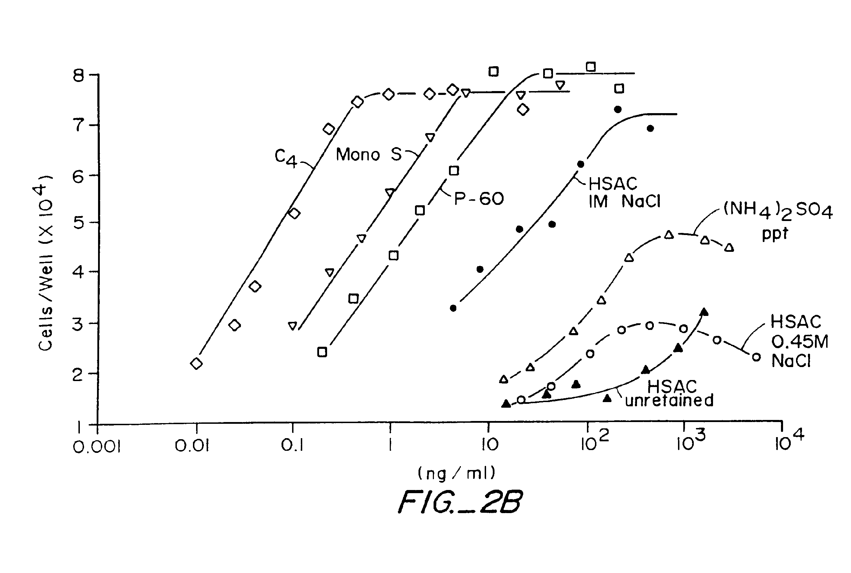

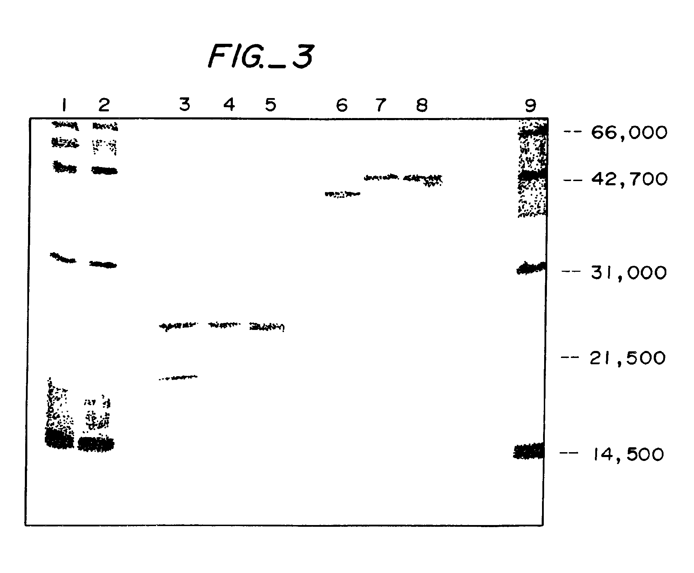

Final purification of the endothelial cell mitogenic activity was achieved by RP-HPLC with a C₄ Vydac column (Fig. 2A), a preparative method suitable for amino acid sequence analysis. Although losses in biological activity were encountered, presumably because of the acid conditions and solvent used, these were not serious enough to prevent the detection of bioactive fractions. All of the bioactivity detected was present in two closely apposed sharp peaks of protein which, when analyzed by Na₂ Dod S0₄/PAGE gave the same single band on the silver stained gel (Fig. 3). The RP-HPLC-C4 step resulted in at least a seven fold increase in specific biological activity with a recovery of 50% (Table 1). Fig. 2B illustrates the relative potency of the various fractions at different stages of purification.

When the bioactive 0.23 M and 0.28 M NaCI pooled Mono S fractions were chromatographed under similar conditions on the C₄ column, a biological profile identical to that observed for the 0.33 M pooled Mono S fractions was obtained. The major portion (90%) of the bioactivity coincided with two closely apposed peaks of proteins which eluted in the same position as those observed with the 0.36 M NaCI Mono S fraction. When analyzed by Na₂ Dod S0₄/PAGE these peaks gave, under reduced condition, a common band at 23 kDa, migrating in the same position as that observed in Fig. 3.

### Physical and Biological Characterization of the Growth Factor

The purified factor when run under unreduced condtions had an estimated molecular weight of 46 kDa (Fig. 3). This value is in good agreement with its elution position on the sizing Bio Gel P-60 column run in solvents expected to maintain the native conformation. When run under reduced conditions the apparent molecular mass was 23 kDa (Fig. 3). From these data, it appears that the mitogen consists of two polypeptide chains with molecular mass of 23 kDa. Given that a single N-terminal sequence was obtained, the dimeric molecule is probably composed of two identical or at least very homologous chains.

The apparent indistinctness of the 46 kDa band could be intepreted as indicating the presence of a glycoprotein. In order to explore this point the bioactive HSAC fractions were applied on a concanavalin A Sepharose column in 10 mM Tris pH 7.3, 0.05 M NaCl, 5mM MgCl₂. All of the biological activity was retained by the column. Elution with high salt (0.5 M NaCI) did not elute any significant amount of bioactivity, while elution with 10 mM methylmannoside did result in the recovery of 25% of bioactivity applied on the column. Elution with 0.2 M methylmannoside did not result in further recovery of bioactivity. These results suggest that the factor is a glycoprotein with strong affinity for concanavalin A. However, the poor biological recovery of the factor from that type of affinity chromatography resin makes concanavalin A unsuitable as a step of purification.

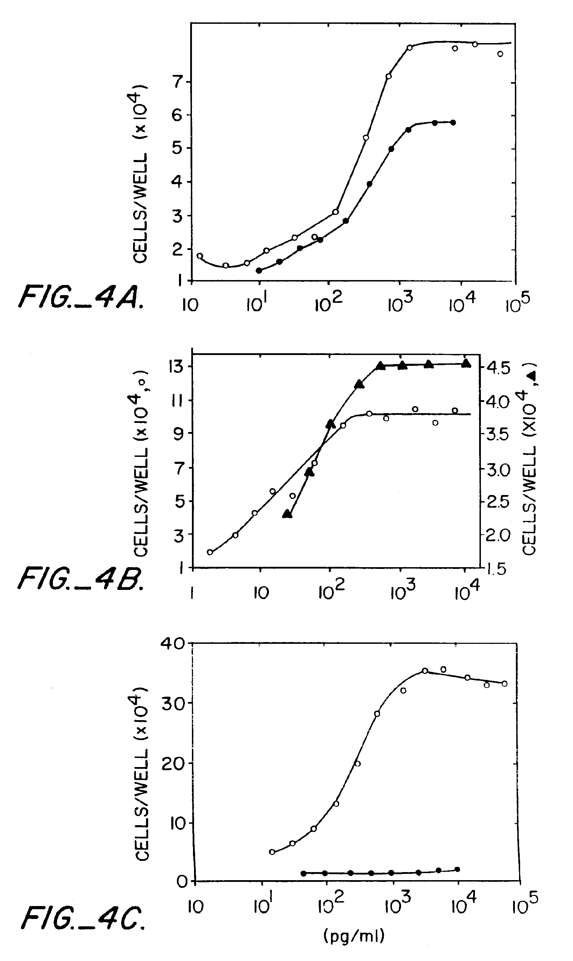

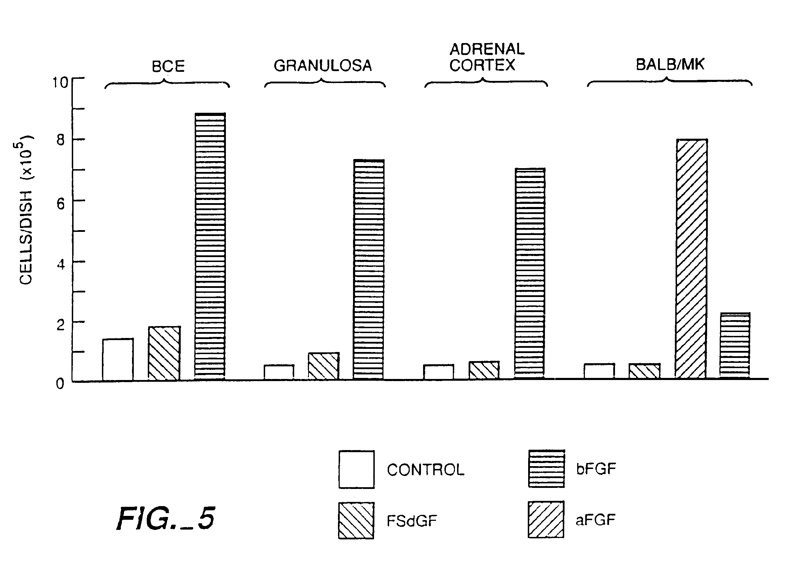

The dose response curves for the growth factor depicted in Figs. 2B and 4 illustrate that as little as about 25 pg/ml stimulates ACE proliferation. Saturation was observed at about 500 pg/ml with an ED ₅₀ of 65 pg/ml (Fig. 4B). These values compared favorably with the range of concentrations where bFGF promotes the proliferation of ACE cells (minimal effect at 10 pg/ml, saturation 200 pg/ml, and ED ₅₀ at 50 pg/ml, ref. 22 and Fig. 4B). However, the final density of the culture grown in presence of the FS derived growth factor was half that of cultures exposed to optimal concentrations of bFGF. Nevertheless, if one considers that the MW of the FS cell derived growth factor is 2.5 times that of bFGF, this new factor has essentially the same potency on a molar basis as bFGF. In addition to its ability to stimulate the proliferation of ACE cells the FS derived growth factor stimulated the growth of bovine brain derived capillary endothelial cells as well as that of HUE cells (Fig. 4A). These results indicate that the mitogenic effect of the factor is not limited by species variation nor by the origin of the vascular endothelial cells. However, and in contrast with bFGF, the factor is not mitogenic for BHK-21 cells (Fig. 4C), nor is it mitogenic for adrenal cortex cells, corneal endothelial cells, granulosa cells or BALB/MK cells (Fig. 5) Therefore, and in contrast with FGF, this factor seems to have a unique specificity for vascular endothelial cells.

### Detailed Description of Figures 1-7

### Fig. 1. Purification of FSdGF by HSAC. gel exclusion chromatography and Mono S ion exchange chromatography

Fig. 1 A. Approximately 350 ml of the (NH₄)₂ S0₄ precipitate fractions derived from 21 liters of FS cell-conditioned medium and dialyzed against 10 mM Tris HCI pH 7.3, 50 mM NaCl, were loaded onto a heparin Sepharose column (1.5 cm x 12 cm, 25 ml bed volume) at a flow rate of 150 ml/hr. The column was then washed with 150 ml of the equilibration buffer (20 mM Tris-HCI pH 7.3, 50 mM NaCI), and the retained proteins (50% of the total protein applied on the column) were eluted with a stepwise application of increasing NaCI concentrations (0.15 M, 0.45 M, 1 M and 3 M NaCI.). Fraction size was 2 ml, and the flow rate was 60 ml/hr. Chromatography was performed at 4°C and absorbancy was monitored at 280 nm. The histogram and open circles show the relative ability of the different pooled or individual fractions to stimulate the proliferation of low density ACE cell clutures (5 x 10³ cells/35mm dish). In the case of the pooled fractions (elute, wash and 0.15 M NaCI), aliquots were diluted ten fold in 0.2% gelatin in PBS and 10 µl aliquots were bioassayed. In the case of the individual 0.45 M and 1 M NaCl fractions, aliquots were diluted one hundred fold in 0.2% gelatin in PBS, and 10 µl aliquots were bioassayed. The majority of the biological activity was present in the 1 M NaCI eluate.

Fig. 1 B. After concentrating the 1 M NaCl HSAC bioactive fractions 126 to 133 to 1 ml in an Amicon YM10 concentrator, the ultrafiltration retentate was applied on a Bio Gel P-60 column (100-200 mesh, 1 x 95 cm) equilibrated and run at 4°C in PBS. The flow rate for development of the column was 6 ml/hr, and 1.45 ml fractions were collected. Absorbancy was monitored at 280 nm. The elution positions of molecular mass markers (in kDa) were as indicated by the arrows. Aliquots of each fraction from the column were diluted 1 to 100 in 0.2% gelatin in PBS, and 10 µl aliquots were bioassayed in ACE cells in 12 well dishes, as described in Material and Methods. Most of the bioactivity eluted as a single peak with an apparent MW of 40 to 45 kDa.

Fig. 1 C. The bioactive fractions 26 and 29 eluted from the Bio Gel P-60 column were pooled and diluted three fold with 20 mM HEPES pH 8.3. Using a 50 ml Super loop, the sample was then applied on a Mono S HR 5/5 column equilibrated in the 20 mM HEPES pH 8.3 room temperature. The column was eluted with a multilinear gradient of NaCI (0 M to 1 M) as follows: 0 M NaCI for 5 min, 0 M NaCI to 0.45 M NaCI in 45 min, 0.45 M NaCl to 1 M NaCI in 15 min, 1 M NaCl for 5 min. Absorbancy was monitored at 280 nm. Flow rate was 1 ml per min and 1 ml fractions were collected. Aliquots of each fraction were diluted 1 to 100 in 0.2% gelatin in PBS, and 10 µl aliquots were bioassayed on ACE cells in 12 well dishes as described in the Materials and Methods section. The histograms show the distribution of the biological activity with most of the biological activity eluting in fractions 37 to 40 (0.33 M NaCI). Fractions indicated by the asterisks were pooled and further examined by RP-HPLC using C₄ column.

Fig. 2 Reverse phase HPLC of the Mono S purified and bioactive fractions and comparison of the ability of FS cell conditioned medium at various stages of purification to stimulate the proliferation of low density ACE cell cultures.

Fig. 2A. The active Mono S fractions (fraction 38 to 40; Fig. 3) were loaded onto a Vydac C₄ column (25 x 0.46 cm, 5 µm particle size, 300 A pore size) equilibrated in 0.1% (v/v) TFA, 20% acetonitrile. The arrows shown the times of injection. Protein was eluted with a 115 min linear gradient of 20-45% acetonitrile in 0.1% TFA at a flow rate of 0.6 ml/min, at room temperature. Fractions of 1.5 ml were collected except in the region where the bioactivity was expected to elute; in this region fraction volumes were limited manually to the size of the individual peak fractions. Aliquots of each fraction were diluted 1 to 10 with 0.2% gelatin in PBS and bioassayed as described in material and methods. The histogram shows the distribution of the biological activity. The peak fractions (fractions 25, 26) indicated by the asterisks were used individually for structural studies and further analysis of their biological activity.

Fig. 2B. Low density ACE cell cultures (5 x 10³ cells/well) were seeded and their proliferation was measured as described in Material and Methods. Samples tested were (NH₄)₂ S0₄ precipitate [Δ]; pool of the HSAC 1 M NaCl fractions [▲]; pool of the bioactive Bio Gel P-60 fractions [0]; pool of the bioactive Mono S fractions [□]; bioactive C₄ fraction [0]. Individual points are the mean of triplicate determinations, and standard deviations were less than 10% of the mean. Control cultures exposed to DMEM supplemented with 10% CS had a final cell density of 1.5 x 10⁴ cell/well.

Fig. 3 NaDodSO₄/PAGE of the bioactive fractions purified by C₄ RP-HPLC.

The fractions in each of the three peaks of activity from Mono S ion-exchange chromatography step (eluting at 0.23M, 0.28M, and 0.33M NaCI, respectively; see Fig. 1 C) were pooled, and each pool was further purified by C4 RP-HPLC, as described in Fig. 2A. In each case, the two peaks of activity eluting from the C4 column (see Fig. 2A) were collected and pooled. Samples of the pooled material from each of the three runs of the C4 column were then subjected to electrophoresis on a 15% polyacrylamide gel, under reducing (lanes 3 - 5) or non-reducing (lanes 6 - 8 conditions. The gel was then stained using the silver nitrate stain kit of BioRad. The samples were: lanes 3 and 6, 0.23 M pool; lanes 4 and 7, 0.28M pool; lanes 5 and 8, 0.33M pool. The molecular weight markers runs in lanes 1, 2 and 9 were: bovine serum albumin (MW 66,000), ovalbumin (MW 42,700), carbonic anhydrase (MW 31,000), soybean trypsin inhibitor (MW 21,500), and lysozyme (MW 14,500).

Fig. 4. Comparison of the ability of pituitary derived bFGF versus FSdGF to stimulate the growth of HUE cells (A), ACE cells (B), and BHK-21 cells (C).

Fig. 4A. Low density cultures of HUE cells (5 x 10³ cells per 1 cm diameter gelatinized well) were exposed to HEPES (25 mM) buffered medium 199 supplemented with 100 µg.ml heparin, 10-⁸M selenium, 20% FCS and increasing concentrations of either pituitary derived bFGF (0) or FSdGF(•). Heparin was added only once at the time of seeding while both bFGF and FSdGF were added every other day. After 6 days in culture, triplicate wells were trypsinized and cell counted. The final desnity of cultures exposed to 20% FCS alone was 1.5 x 10⁴ cells per well. Standard deviation was less than 10% of the mean.

Fig. 4B. Low density cultures of ACE cells (5 x 10³ cells per 1 cm diameter well) were exposed to DMEM supplemented with 10% CS and increasing concentrations of either pituitary derived bFGF (0) or FSdGF(•) added every other day. After 5 days in culture, triplicate wells were trypsinized and cell counted. The final density of cultures exposed to 10% CS alone was 1.3 x 10⁴ cells per well. Standard deviation was less than 10% of the mean.

Fig. 4C. Low density cultures of BHK-21 cells (2 x 10⁴ cells per 35 mm gelatinized culture dishes) were exposed to 2 ml of DMEM-F12 (1 to 1 v/v) supplemented with 2.5 µg/ml Fungizone, 50 µg/ml gentamicin, 10 µg/ml transferrin, 5 µg/ml insulin and increasing concentrations of either pituitary derived bFGF (0) or FSdGF (e). Insulin and transferrin were added only once, bFGF and FSdGF were added every other day After 4 days in culture triplicate dishes were trypsinized and cell counted. The final density of culture exposed to transferring and insulin alone was 1.38 x 10⁴ cells/plate. Standard deviation was less than 10% of the mean.

Fig. 5Comparison of the ability of bFGF versus FSdGF to stimulate the proliferation of BCE cells, granulosa cells, Adrenal cortex cells and BALB/MK cells.

2 x 10⁴ BCE cells, granulosa cells, or adrenal cortex cells per 35 mm dishes were seeded in the respective media (DMEM supplemented with 10% CS for BCE and adrenal cortex cells and F-12 medium supplemented with 2.5% CS for granulosa cells). BALB/MK cells were seeded at a density of 3 x 10⁴ cells per 35 mm dishes in low Ca modified Eagle medium (40) supplemented with 10% FCS. Pituitary derived bFGF (bFGF, 2ng/ml) or FSdGF (5ng/ml) were added every other day. BALB/MK cells were also exposed to aFGF (1 Ong/mi) since this mitogen is as potent as EGF in promoting their growth. After 6 days in cultures, cells were trypsinized and counted in a Coulter counter. Standard deviation was less that 10% of the mean.

### Microsequencing Reveals a Unique N-Terminal Amino Acid Sequence

A single amino acid was identified in each of the first 12 cycles, consistent with a homogeneous protein. The yield of the amino terminal residue was 150 picomoles. Unambiguous assignments made for cycles 1 to 12 were as follows: Ala-Pro-Met-Ala-Glu-Gly-Gly-Gln-Lys-Pro-His-Glu. A search of the NBRF database using the FASTP program of Lipman and Pearson (35) found no significant homology between this sequence and any known protein.

### Sequence Analysis of Tryptic Peptides

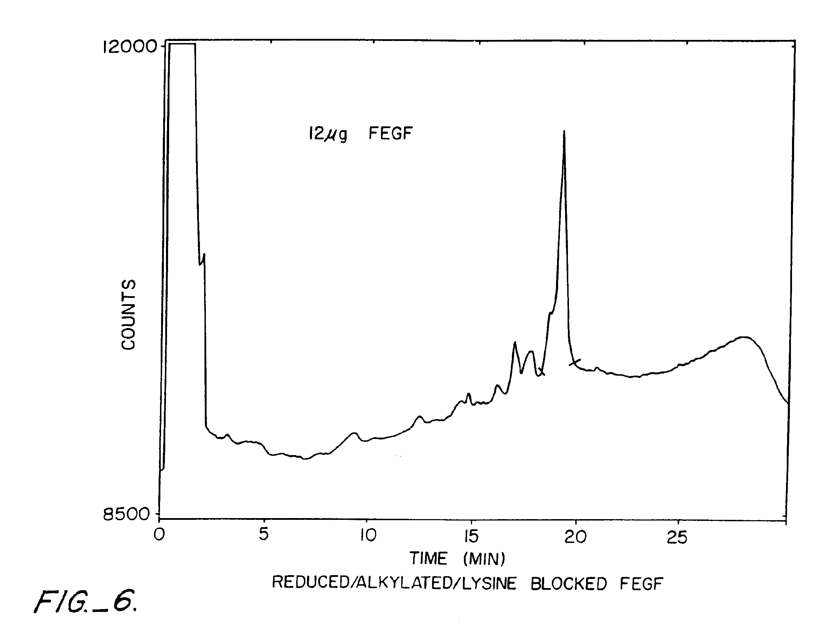

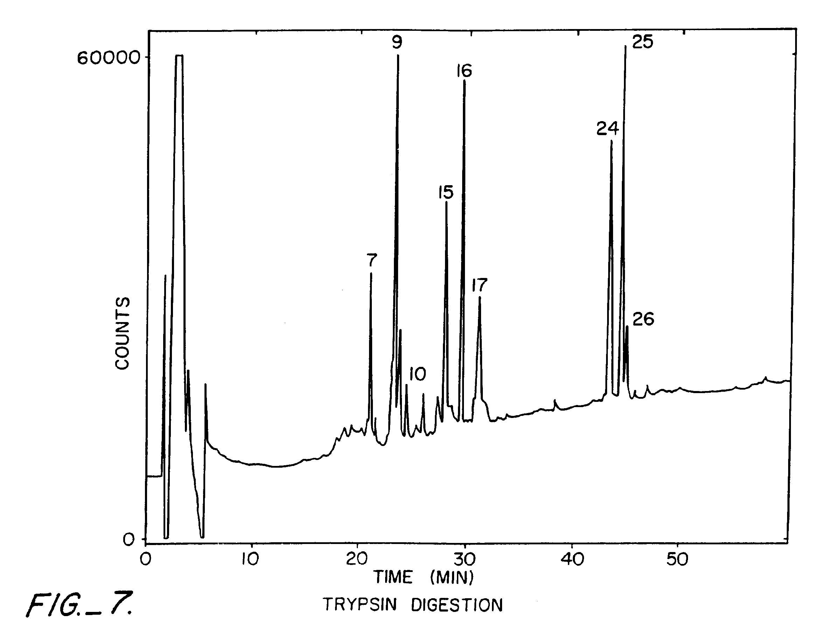

12 µg of FSdGF was reduced in 200 µl of a solution containing 6M guanidine hydrochloride, 0.5M Tris base pH=8, 1 mM EDTA and 10mM dithiothreitol for one hour at 37°C. The reduced protein was alkylated by the addition of solid iodoacetamide to give a final concentration of 25mM and the reaction allowed to proceed for 30 minutes at room temperature. Following the alkylation reaction lysines were modified with succinic anhydride. Four aliquots of µl each freshly prepared 100mg/ml succinic anhydride in acetonitrile were added at five minute intervals. After the final addition the protein was desalted by reverse phase HPLC using a Hewlett-Packard 1090L HPLC equipped with a diode array detector and a 0.46x3 cm Brownlee Labs C₄ (BU 300) column. Solvent A was 0.1% TFA in water, solvent B was 0.1% TFA in acetonitrile. The gradient was 20-60% B over 30 minutes at 0.5 ml/min flow rate. The elution profile was monitored at 214 nm and the single protein peak was collected by hand. The chromatogram is shown in Figure 6. After drying on a Speed-Vac (Savant) the protein redissolved in 200µl of 100 mM ammonium bicarbonate. 0.4 mg of TPCK treated trypsin was added and the sample was incubated for an additional 2 hours at 37°C. An additional 0.4 µg of trypsin was added after 2 hours and the sample incubated for an additional 2 hours at 37°C. Peptides were separated on the same HPLC using the same solvents as described above. The column was a 0.21 x 15 cm Vydac C₁₈ (218TP5215) run at 0.25 ml/min. The HPLC program initially flowed for 5 minutes at 10% B to and then ran a linear gradient fo 10 to 70% B over 65 minutes. The elution of peptides was monitored at 214 nm and peaks were collected by hand. The chromatogram is shown in Figure 7. For amino acid sequence analysis peaks 7, 15, 16, 24 and 25 were spotted and dried directly on the glass fiber discs used as supports in the protein sequencer. No sequences were obtained from peptides 15 and 16. Peptide 7 gave a mixed sequence with the major sequence H-lle-X-Pro-His-Gln-Ser-Gln-His-Ile-Gly-Glu-Met-Ser-Ile-Leu-Gln-His-Asn- and the minor sequence H-X-Val-Leu-Asp/Phe-Val-Val-X-X-Pro-. Peptide 24 gave a single sequence H-Ser-Phe-Cys-Arg-Pro-lle-Glu-Thr-Leu-Val-Asp-lle-Phe-Gln-Glu-Tyr-Pro-Asp-Glu-lle-. Peptide 15 gave a single sequence H-Ser-Phe-Cys-Arg-Pro-lle-Glu-Thr-Leu-Val-Asp-lle-Phe-Gln-Glu-Tyr-Pro-Asp/lle-Glu. Ser 1 of the peptides 24 and 25 corresponds to Ser 23 of the amino terminal sequence shown above so that these sequences can be merged to give the sequence of the first 42 aminio acids of the protein.

Unambiguous assignments made for cycles 1 to 12 were as follows: ¹Ala-Pro-Met-Ala-Glu-Gly-Gln-Lys-Pro-His-Glu. A search of the NBRF database using the FASTP program of Lipman and Pearson (35) found no significant homology between this sequence and any known protein.

### Compositions and Uses

FSdGF provided by the invention is useful as a wound healing agent, particularly in applications where it is desired to re-endothelialize vascular tissue, or where the growth of a new capillary bed (angiogenesis) is important.

FSdGF can, therefore, be used in the treatment of full-thickness wounds such as dermal ulcers, including the categories of pressure sores, venous stasis ulcers and diabetic ulcers. In addition, FSdGF can be used in the treatment of full-thickness burns and injuries site for a skin graft. In this case, the FSdGF is either applied directly to the site or is used to soak the skin that is being transplanted prior to grafting. In a similar fashion, FSdGF will be used in plastic surgery when the reconconstruction is required following a burn, other trauma or for the cosmetic purposes.

Angiogenesis is also important in keeping wounds clean and non-infected. FSdGF can, therefore, be used in association with general surgery and following the repair of cuts and lacerations. It is particularly useful in the treatment of abdominal wounds where leakage of fecal material increases in the risk of infection. Neovascularization is also key to fracture repair since blood vessels develop at the site of bone injury. Adminstration of FSdGF to the site of a fracture is, therefore, another utility.

In the cases where FSdGF is being used for topical wound healing, as described above, it may be administered by any of the routes described below for the reendothelialization of vascular tissue, or more preferably by a topical means. In these cases, it will be administered as either a solution, gel, cream, ointment or as a dry powder directly to the site of injury. Slow release devices direction FSdGF to the injured site will also be used. In topical applications, FSdGF will be applied at concentration ranging from 50 to 1,000 µg/ml either in a single application, or in dosing regimens that are daily or every few days for a period of one to several weeks.

FSdGF can be used as a post-operative wound healing agent in balloon angioplasty a procedure in which vascular endothelial cells are removed, together with atherosclerotic plaques. FSdGF can be applied to vasuclar endothelial surfaces by systemic or local intravenous application either as intravenous bolus injection or infusions. If desired, the FSdGF can be administered over time using a micrometering pump. Suitable compositions for intravenous administration comprise FSdGF in an amount effective to promote endothelial cell growth and a parenteral carrier material. The FSdGF can be present in the composition over a wide range of concentration, for example, from about 50 wg/ml to about 1,000 µg/ml using injections of 3-10 ml per patient. Any of the known parenteral carrier vehicles can be used, such as normal saline or 5-10% dextrose.

FSdGF may also be used to promote endothelialization in vascular graft surgery. In the case of either vascular grafts using transplanted vessels of synthetic material, for example, FSdGF can be applied to the surfaces of the graft and/or at the junctions of the graft and the existing vasculature in order to promote the growth of vascular endothelial cells. For such applications, the FSdGF may be applied intravenously as described above for balloon angioplasty or it may be applied directly to the surfaces of the graft and/or the existing vasculature either before or during surgery. In such cases, it may be desired to apply the FSdGF in a thickened carrier material so that it will adhere to the affected surface. Suitable carrier materials include, for example, 1-5% carbopol. The FSdGF may be present in the carrier over a wide range of concentrations, for example, from about 50 µg/mg to about 1000 µg/mg.

FSdGF may also be employed to repair vascular damage following myocardial infarction. The FSdGF is administered intravenously for this purpose, either in individual injections or by micrometering pump over a period of time as described above.

FSdGF may also be used as a growth factor for the in vitro culturing of endothelial cells. For such uses, FSdGF can be added to the cell culture medium at a concentration from about 10 ng/ml to about 10 µg/ml.

The amino acid sequence of FSdGF will be used to design synthetic oligonucleotide probes for the retrieval of the FSdGF gene. These probes will either be of a mixed sequence based on all possible genetic code choices, or will be of single sequence based on codon choice preferences and other factors. In the first instances, probes based on the amino acid sequence of bovine FSdGF will be used to screen either bovine cDNA libraries made from folliculostellate cells, or bovine genomic libraries. Bovine DNA clones encoding FSdGF thus isolated will be sequenced to determine the complete coding and hence amino acid sequence of bovine FSdGF. The bovine FSdGF clones will then be used as probes to isolate human FSdGF sequences from either cDNA libraries generated from tissues shown to express the factor, or from human genomic libraries. In this way, the complete nucleotide and hence amino acid sequence of human FSdGF can be established.

### Discussion

This is the first identification, purification and biological characterization of a novel heparin-binding endothelial cell growth factor (VEGF) from culture media conditioned by pituitary FC. Additional detail is provided in the Examples below.

### Results

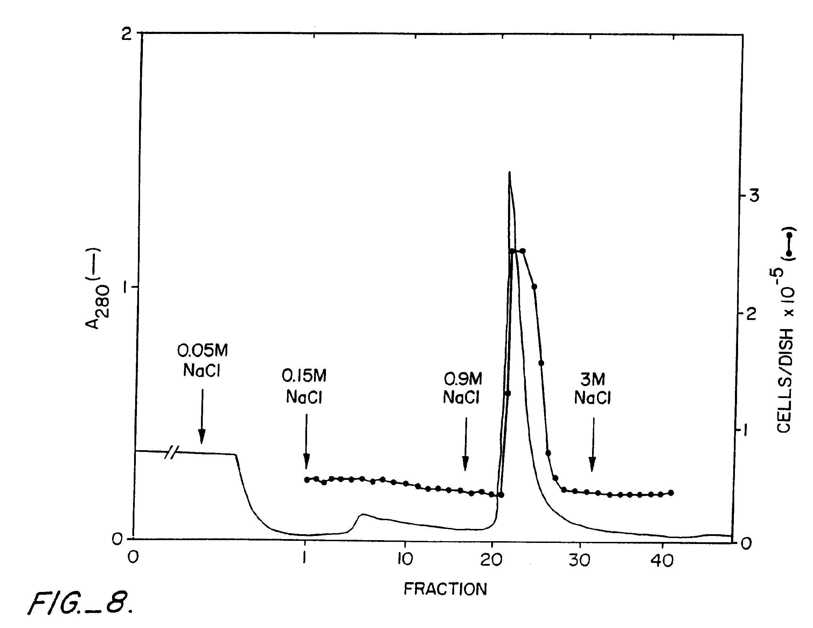

The media conditioned by FC was found to stimulate the proliferation rate of low-density microvascular endothelial cells. Table 2 summarizes the steps for the purification of the growth promoting activity and the corresponding yield in bioactivity. The mitogenic activity was precipitated by 50% ammonium sulfate and resuspended to a volume suitable for subsequent purification. The H-S step provided an efficient way of further concentrating such activity and also provided a ten fold purification. Approximately 90% of the biological activity was eluted in the presence of 0.9 M NaCI (Fig. 8). The bioactivity was not affected heating the fractions at 65° C for 5 min and was decreased 25-30% following the exposure to 0.1% TFA (pH 2) for two hours.

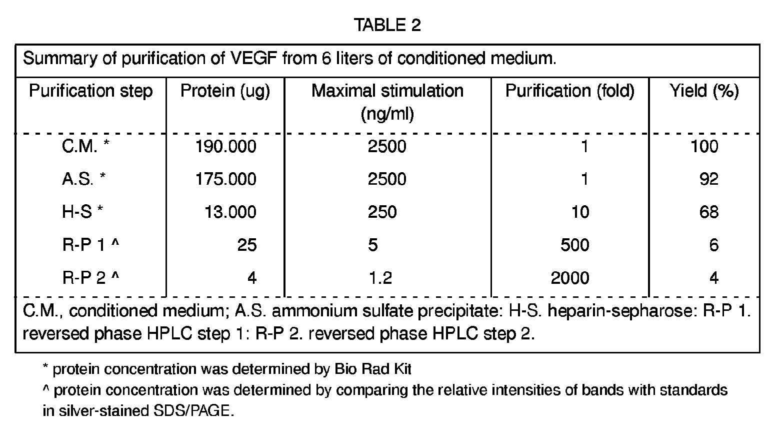

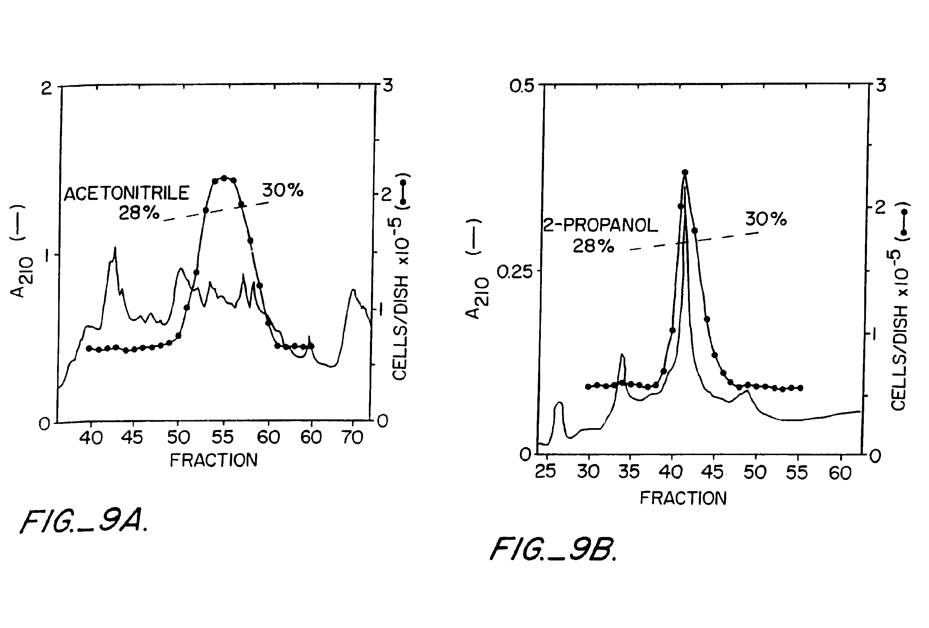

The most bioactive HS fractions was applied to a semi preparative C4 reversed phase HPLC column, a method suitable for rapid purification of proteins and peptides. The bioactivity was eluted as a single peak in the presence of about 29% acetonitrile (Fig. 9A). A silver-stained (56) SDS/PAGE gel on the most bioactive fractions revealed the presence of three or four bands. These fractions were further purified by a second reversed phase HPLC step, using an analytical C4 column which was eluted with a gradient of 2-propanol, instead of acetonitrile. A single peak of bioactivity corresponding to a distinct peak in the absorption profile was obtained. (Fig. 9B).

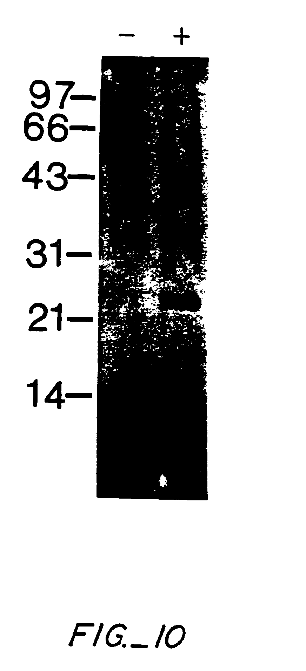

The peak fractions from the second reversed phase step displayed a single band on a silver stained SDS/PAGE, with an apparent Mᵣ of about 23 kDa under reducing conditions (Fig. 10). The intensity of staining of the band was highly correlated to the mitogenic activity across the bioactivity profile. Because previous experiments, using a molecular sieve with a TSK G 3000 SW column, suggested a Mᵣ in the range of 40-43 kDa, the possibility that the growth factor in native conditions is a dimer was considered. This was strongly suggested by the finding that the purified material had an apparent Mᵣ of about 45 kDa in a silver stained SDS-PAGE under non-reducing conditions (see Fig. 10).

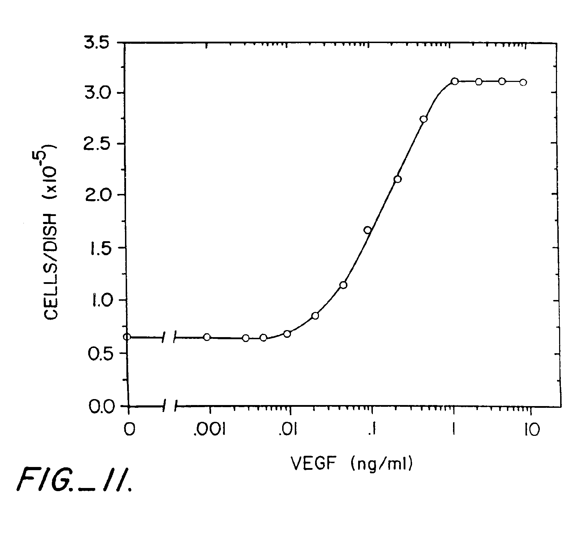

As illustrated in Fig. 11, the dose response curve for the purified growth factor revealed a half maximal effect on adrenal cortex-derived capillary endothelial cells proliferation at 150-200 pg/ml and a maximal effect at 1-1.5 ng/ml. These values were derived from protein sequencing and were found to be in good agreement with those obtained by comparing the relative intensitieis of bands with standards in silver stained SDS/PAGE.

Gas phase microsequencing of the purified material demonstrated unambiguously a single N-terminal amino acid sequence. The first five residues are ¹Ala-Pro-Met-Ala-Glu. Another way to describe the N-terminal amino acid sequence is Ala-Pro-Met-Ala-Glu-Gly-Gly-Gln-Lys-Pro-His-Glu-Val-Val-Lys-Phe-Met-Asp-Val-Tyr-Gln-(Arg)-Ser-Phe-X- Arg-Pro-Ile-Glu-Thr-Leu-(Val)-X-Ile-X-(Gln)-Glu-Tyr-(Pro)-where the amino acids in parenthesis are known with a high degree of certainty and -X- denotes an amino acid of as yet unknown identity A computer search revealed that such a sequence does not display significant homology to any previously known protein.

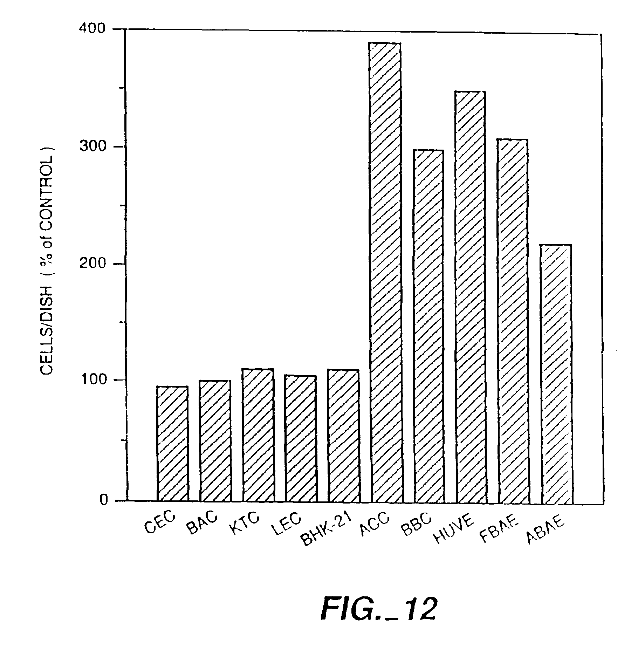

The bioactivity of the growth factor was also tested with different cell types. As shown in Figure 12, appreciable activity was observed only in cell types of vascular endothelial origin, such as fetal and adult bovine aortic endothelial cells, bovine brain capillary endothelial cells and human umbilical vein endothelial cells. In contrast, adrenal cortex cells, lens epithelial cells, corneal endothelial cells, BHK-21 fibroblasts and keratinocytes failed to show any significant mitogenic response.

The growth factor was purified by using a combination of ammonium sulfate precipitation, H-S affinity chromatograph and two reversed phase HPLC steps. Analysis of the purified material by SDS PAGE reveal a Mᵣ of about 45 kDa under non reducing conditions. When the material was analyzed in the presence of 2-mercaptoethanol, a single band with a Mᵣ of 23 kDa was visualized, indicating that the growth factor is a dimer composed of two subunits of identical apparent molecular weight. Microsequencing of the purified material reveals a unique N-terminal amino acid sequence.

The growth factor was heat and acid stable and its p. I., as estimated by chromatofocusing on a Mono P column is about 8.5.

The purified growth factor was able to stimulate the proliferation of vascular endothelial cells at concentrations between 25 pg and 1-1.5 ng/ml. These values, assuming a Mᵣ of 45 kDa, correspond respectively to 0.55 pM and 22-33 pM. Such values are in the same range as those obtained with bFGF (2,56). However, the novel growth factor did not induce any appreciable mitogenic effect on corneal endothelial cells, lens epithelial cells, BHK-21 fibroblasts, adrenal cortex cells, or keratynocytes. In contrast, bFGF and aFGF are both potent mitogens for all of these cell types (2,56).

The ability of VEGF to bind heparin may have implications as to its in vivo function and regulation. Heparin sulphates are fundamental components of the extracellular matrix and have been proposed to play a crucial role in determining contact between target cells and heparin-binding growth factors (16,57,58,59).

The presence of VEGF in pituitary FC indicates a role for these cells in the development, organization and maintenance of a differentiated state of the complex microvasculature of the adenohypophysis.

It is presently unknown whether VEGF is expressed in organs other than the pituitary gland. However, considering the fundamental role of vascular endothelial cells growth and angiogenesis in a great variety of normal and pathological proliferations (60), it is expected that the distribution of the growth factor is widespread. With this context, it is of interest that PDGF, EGF, TGF alfa, TGF beta, FGF, NGF, which were initially believed to be restricted to specific cells or tissues, were later found to have a much broader and sometimes ubiquitous distribution (61).

The genes for bFGF and aFGF, the best characterized endothelial cell mitogens, do not code for a conventional signal peptide (17, 43). Accordingly, these growth factors appear to be sequestered inside the cells of origin and apparently do not have direct access to target cells (2, 15,45). It has been suggested that bFGF may be incorporated into the basement membrane and be subsequently released in a soluble form only when the matrix is degraded following the action of specific enzymes (16). Such a mechanism of release suggests a role for the growth factor mostly or exclusively in events which involve degradation of the basement membrane or cell lysis, such as organ remodeling, wound healing or neoplasia (60).

In contrast, a soluble endothelial cell growth factor such as VEGF may play a more dynamic role in the physiological regulation of the vascular endothelial cells proliferation, either in the cyclical growth of blood vessels which takes place in organs such as the corpus luteum (62) or in the tonic maintenace of the differentiated stage of the endothelium in the vascular tree.

Unlike bFGF or aFGF, which are active on a very broad spectrum of cells (2,56), VEGF appears to be specific for vascular endothelial cells. VEGF is special therapeutic significance for conditions in which a selective action on the vascular endothelial cells, in the absence of excessive connective tissue proliferation, is desirable, such as diabetic ulcers or traumatic vascular injuries.

### Detailed Description of Figures 8 to 12

Figure 8 -- Heparin-sepharose (H-S) bioactivity profile of FC conditioned medium. The medium (6 liters) was concentrated and applied to a HS Col. which had been preequilibrated in 10 mM Tris/CI, pH 7.2 containing 50 mM NaCI. The column was washed with the same buffer and then eluted sequentially with 10 mM Tris/Cl, pH 7.2, containing 0.15, 0.9 nd 3 M NaCI. Aliquots of the collected fractions were diluted 100 fold in 0.2% gelatin in PBS 5 µl/ml were applied to capillary endothelial cells for bioassay.

Figure 9A and 9B -- Sequential reversed phase HPLC profiles of endothelial cell mitogenic activity The most bioactive H-S fractions were applied to a C4 column (10 x 250 mm) preequilibrated with 0.1% TFA/20% acetontrile (Fig. 9A). After the column was washed with 10 ml of equilibration buffer, the sample was eluted with a linear gradient of acetonitrile. Aliquots of each fraction were diluted tenfold with 0.2% gelatin in PBS and 5 wl/ml were applied to capillary endothelial cells for bioassay The most bioactive fractions were pooled and applied to a C4 column (4.6 x 250 mm) which had been preequilibrated with 0.1% TFA/20% 2-propanol (Fig. 9B). After washing the column with 3 ml of equilibration buffer, the sample was eluted with a linear gradient of 2-propanol. Aliquots of fractions were tested for bioactivity

Figure 10 -- NaDodS0₄/PAGE analysis of most bioactive fraction from chromatogram shown in Fig. 9B. Two 50 µl aliquots of such fraction were dried in a speed vac and redissolved in sample buffer containing (+) or not (-) 2,5% 2-mercaptoethanol. The samples were heat-denatured and electrophoresed in a 12.5% PAGE which was subsequently silver stained. The molecular weight markers are: phosphorylase B, 97,400; bovine serum albumin, 66,200; ovalbumin, 43,000; carbonic anhydrase, 31,000; soybean trypsin inhibitor, 21,500; lysozyme, 14,400.

Fiaure 11 -- Dose-responsive growth of adrenal cortex derived capillary endothelial cells in the presence of purified VEGF. Cells were seeded at the density of 1 x 10⁴/well in 12 well plates. The indicated amounts of VEGF were added a few hours after plating in 5 µl/ml aliquots. After five days, cells were counted in a Coulter counter. The results shown represent mean values of three separate experiments conducted in duplicate. Duplicates in each experiment varied less than 10%.

Figure 12 -- Effects of VEGF on the growth of different cell types. CEC, corneal endothelial cells; BAC, bovine adrenal cortex cells; KTC, keratynocytes; LEC, lens epithelial cells; BHK-21, baby hamster kidney cells, clone 21; ACC, adrenal cortex capillary endothelial cells; BBC, bovine brain capillary endothelial cells; HUVE, human umbilical vein endothelial cells; FBAE, fetal bovine aortic endothelial cells, ABAE, adult bovine aortic endothelial cell. Cells were seeded in their respective growth media, incubated with a maximal concentration of VEGF and counted after 4 or 5 days. Results are expressed as a percent of appropriate control.

Angiogenesis is a multi-step phenomenon which involves capillary endothelial cell profileration, migration and tissue infiltration(1). It plays a central role in a variety of physiological and pathological process such as embryonic development, wound healing, atherosclerosis and tumor growth (1,2). Several factors that induce angiogenesis have recently been isolated and characterized. Of these, only the basic and acidic forms of FGF have been shown to directly control all steps of angiogenesis including vascular endothelial cell proliferation, migration and increased expression of plasminogen activator and collagenase activity(2).

Despite the evidence the FGF is angiogenic, two puzzling questions point to the existence of other angiogenic factors which could complement the action of FGF. First, FGF lacks the hydrophobic signal sequences that govern secretion(12,13), yet for acceptance as an angiogenic factor any putative mediator should be shown to be a diffusible substance which induces new capillary formation from a microcirculatory bed. Second, FGF is produced by endothelial cells themselves(14, 15). If FGF is present in and around endothelial cells yet the cells are quiescent, other factors must come into play to trigger angiogenesis.

The present invention also relates to the isolation and characterization of a new endothelial cell mitogen produced and secreted by AtT20 cells. This mouse cell line is available from The America Type Culture Collection, 12301 Park- lawn Drive, Rockville, Maryland. The factor produced by AtT20 cells has a unique target cell specificity, since it stimulates only vascular endothelial cells to proliferate and does not affect other cell types sensitive to FGF.

This factor was discovered during studies on expression of bFGF and possible secretory pathways the pituitary cell line AtT-20(5). The cells were transfected with a chimeric bFGF gene composed of the coding sequence for the growth hormone secretion signal peptide fused with the coding sequence for the bFGG gene(6). The murine cell line was selected because it has retained normal secretory functions and can be used to study the molecular events involved in packaging and secretion of protein(5). It was hoped that the bFGF expressed would have been secreted by these cells either through a constitutive secretory pathway or through a secretory pathway involving secretory granules. When medium conditioned by transfected AtT-20 cells were examined for angiogenic activity, a considerable amount of activity was present which could not be immunoneutralized by antibodies against bFGF and which did not crossreact in a (RIA) specific for bFGF. Likewise, media conditioned by parental cells which did not express the bFGF gene also contained a considerable amount of bioactivity, suggested that a factor unrelated to bFGF was responsible for it.

The endothelial cell mitogen present in the conditioned medium was then purified by a combination of steps including heparin sepharose affinity chromatography (HSAC), exclusion gel chromatography on Sephadex G 100, cation exchange on chromatography on Mono S resin, and finally, by PR-HPLC with a C4 Vydac column (see Table 3). Fractions were assayed for bioactivity using adrenal cortex derived capillary endothelial cells. When the collected material was applied on HS under the conditions described in Fig. 13A the activity eluted in a broad peak in the range of 0.5 to 0.6M. In order to concentrate it, elution with 0.8 M NaCI was carried. This material when applied on Sephadex G 100 gave a major peak of bioactivity which eluted with an apparent MW of about 45kDa (Fig. 13B). When chromatographed on Mono S under the conditions indicated Fig. 13C, a single, major, bioactive peak was observed eluting at 0.28 M NaCI. Final purification was achieved by RP-HPLC using a C4 Vydac column (Fig. 13D). All of the bioactivity detected was present in 2 closely sharp peaks of proteins which when analyzed by Na₂DodS04/PAGE under non reducing conditions gave a single band with an apparent molecular weight of 45 kDa. When seen under reducing conditions, the band had an apparent molecular weight of 23kDa (Fig. 13D). In one of the fractions, a small amount of contaminant with a MW of 27 kDa whose migration was not affected by reduction was also present. This contaminant did not amount to more than 5% of the total. When the major peak activity was rerun under identical condition of a C4 column, a single peak of protein with a small shoulder was obtained (Fig. 13D). Microsequencing of this material reveals a unique terminal amino acid sequence. Approximately 2µg (80 pmole) of protein was sequenced using an Applied Biosystems 477A gas phase protein sequenator. A single amino acid was identified in each of the first 24 cycles consistent with a homogeneous protein. The yield of the amino terminal residue was 30 pmole. Unambiguous assignment modes for cycles 1 to 5 were as follows: Ala-Pro-Met-Ala-Glu. A longer amino acid sequence for the growth factor from AtT-20 was later determined and is described below.

A search of the NBRF database using the FASTP program of Lipman and Pearson(35) found no significant homology between the sequence of the first 22 amino acids and any known protein.

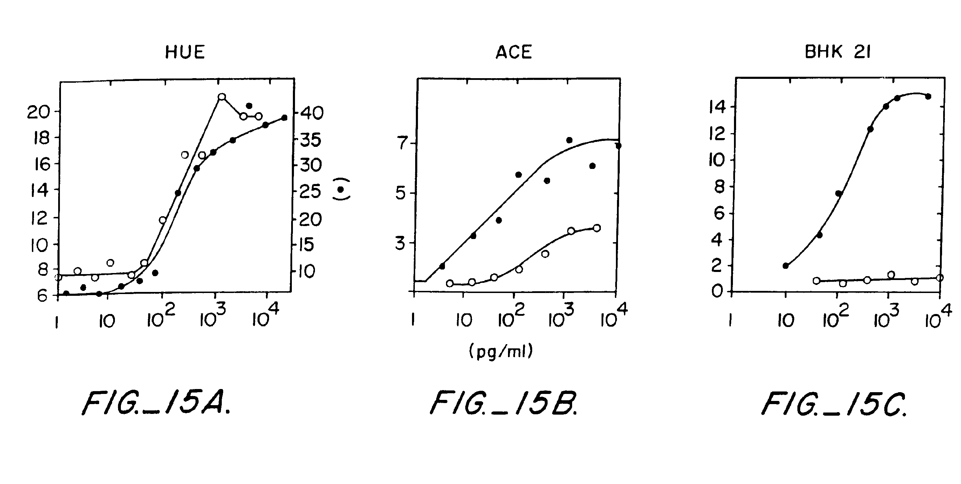

The dose response curve for the growth factor depicted in Fig. 15B illustrates that as little as 50 pg/ml stimulates ACE proliferation. Saturation was observed at 1 ng/ml with an ED₅₀₀f 150 pg/ml. These values compared favorably with the range of concentrations where bFGF promotes the proliferation of ACE cells (minimal effect at about 10 pg/ml saturation at about 200 pg/ml and ED₅₀ at 50 pg/ml,(22) and Fig. 15). However, the final density of the culture grown in presence of the atT20 derived growth factor was half that of cultures exposed to optimal concentrations of bFGF, indicating that the average doubling time of the ACE cells was longer when driven to proliferate by the AtT20 derived growth factor than by bFGF. Nevertheless, if one considers that the MW of the AtT-20 cell derived growth factor is 2.5 times that of bFGF it would indicate that it has about the same potency as bFGF. In addition to its ability to stimulate the proliferation of ACE cells the atT20 derived growth factor did stimulate the growth of bovine brain derived capillary endothelial cells as well as that of HUE cells (Fig. 15A). This indicates that its mitogenic effect was not limited by

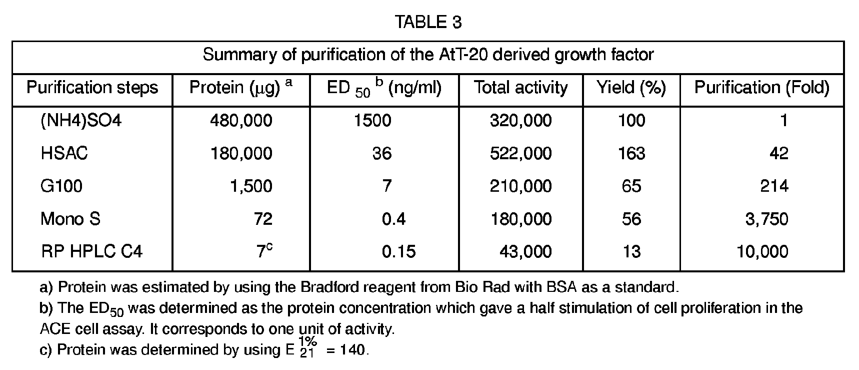

species variation nor by the origin of the vascular endothelial cells. Surprisingly, it did not stimulate the proliferation of even BHK-21 cells, a cell line known to respond to a wide variety of mitogens including TGFa, EGF PDGF, TGFI3 and aFGF or bFGF (as reviewed in (26), nor was it mitogenic for adrenal cortex cells, corneal endothelial cells, granulosa cells, vascular smooth muscle cells or BALB/MK cells (Fig. 15D). Therefore, and in contrast with FGF, it seems to have a unique specificity for vascular endothelial cells. The present data established that AtT20 cells which have retained many important chemical and physiological properties of pituitary corticotroph, in particular the ability to synthesize and release as major secretory products ACTH, B-lipotropin and B-endorphin(64), do also produce an angiogenic factor.

The physical properties of growth factor (MW 45kDa, basic pl, affinity for HS) and biological properties (mitogenic for vascular endothelial cells) indicate that it is distinct from other known growth factors such as EGF, TGFa, PDGF, TGFB or the recently reported keratinocyte growth factor(21). Its lack of recognition by neutralizing polyclonal antibodies directed against aFGF or bFGF as well as its lack of cross reactivity in RIA specific for aFGF or bFGF indicates that it is distinct from FGF. It seems also to be distinct from the recently reported platelet derived endothelial cell growth factor(37) have the same apparent target cell specificity and similar molecular mass, they differ by twenty fold in potency and by their secondary structure, PDECGF being a single chain polypeptide while the AtT20 growth factor has a dimeric structure.

The unique target cell specificity and N-terminal lead to the conclusion that the AtT20 derived growth factor represents a previously unknown growth factor. Although the present study clearly established that this novel growth factor is mitogenic for capillary endothelial cells, it is not yet known whether it can stimulate other events linked to angiogenesis. These include chemotaxis of capillary endothelial cells and activation of the synthesis of cellular enzymes such as collagenase and plasminogen activator which are involved in the breakdown of capillary basement membrane(3). In view of its preferential activity on vascular endothelial cells as compared to other mesoderm derived cells, the name of vasculotropin is suggested for this novel growth factor.

Available structural data should allow studies on the cloning, structure, topology, expression and regulation of the growth factor gene in both physiological and pathological conditions. These studies may provide clues as to its distribution in normal versus malignant tissues as well as to its physiological functions including angiogenesis.

### Detailed Description of Figures 13-16

Fig. 13 Purification at AtT20 growth factor by HSAC, gel exclusion chromatography, Mono S ion exchange chromatography, and R-HPLC on C₄ column
Fig. 13A. Approximately 490 ml of the (NH₄)₂S0₄ precipitate fractions derived from 30 liters (6 collection of 5 liters at 2 day intervals) of AtT-20 cell-conditioned medium (DMEM-H21) supplemented with 5 µg/ml insulin and 10 µg/ml transferrin) and dialyzed against 10 mM Tris HCI pH 7.3, mM NaCl, were loaded onto a heparin Sepharose column (1.5 cm x 12 cm, 25 ml bed volume) at a flow rate of 150 ml/hr. The column was then washed with 150 ml of the equilibration buffer (20 mM Tris-HCI pH 7.3, 50 mM NaCI), and the retained proteins (50% of the total protein applied on the column) were eluted with a stepwise application of increasing NaCI concentrations (0.3 M, 0.8 M and 3 M NaCI). Fraction size was 3 ml, and the flow rate was 6 ml/hr. Chromatography was performed at 4°C and absorbency was monitored at 280 nm. The histogram and closed circles shown the relative ability of the different pooled or individual fractions to stimulate the proliferation of low density ACE cell cultures (5 x 10³ cells/well, 12 well dishes). Conditions for testing were the same as those described in (37). The majority of the biological activity was present in the 0.8 M NaCl eluate.
Fig. 13B. After concentrating the 0.8 M NaCI HSAC bioactive fractions to 1 ml in Amicon YM10 concentrator, the ultrafiltration retentate was applied on a Sephadex G 100 column (1 x 95 cm) equilibrate and run at 4°C in PBS. The flow rate for development of the column (1 x 95 cm) equilibrated and run at 4°C in PBS. The flow rate for development of the column was 6 ml/hr, and 3 ml fractions were collected. Absorbency was monitored at 280 nm. The elution positions of molecular mass markers (in kDa) were as indicated by the arrows. Aliquots of each fraction from the column were diluted 1 to 100 in 0.2% gelatin in PBS, and 10 µl aliquots were bioassayed.
Fig. 13C. The bioactive fractions eluted from the Sephadex G 100 column were pooled and diluted three fold with 20 mM HEPES pH 8.3. Using a 50 ml Super loop, the sample was then applied on a Mono S HR 5/5 column equilibrated in the 20 mM Hepes pH 8.3 at room temperature. The column was eluted with a multilinear gradient of NaCI (0 M to 1 M) as follows: 0 M NaCI for 5 min., 0 M NaCI to 0.45 M NaCl in 45 min, 0.45 M NaCI to 1 M NaCl in 15 min, 1 M NaCl for 5 min. Absorbency was monitored at 280 nm. Flow rate was 1 ml per min and 1 ml fractions were collected. Aliquots of each fraction were diluted 1 to 100 in 0.2% gelatin in PBS, and 10 µl aliquots were bioassyed on ACE cells in 12 well dishes as described above. The histogram shows the distribution of biological activity with most of the biological activity eluting in fractions 33 to 35 (0.28 M NaCI).
Fig. 13D. The active Mono S fractions (fraction 33 to 35; Fig. 13C were loaded onto a Vydac C₄ column (25 x 0.46 cm, 5 µm particle size, 300 A pore size) equilibrated in 0.1% (v/v) TFA, 20% acetonitrile. The arrows show the times of injection. Protein was eluted with a 115 min linear gradient of 20-45% acetonitrile in 0.1% TFA at a flow rate of 0.6 ml/min, at room temperature. Fractions of 1.5 ml were collected. Aliquots of each fraction were diluted 1 to 10 with 0.2% gelatin in PBS and bioassayed as described above. The histogram shows the distribution of the biological activity. The peak fractions (22, 24) were used individually for structural studies and further analysis of their biological activity. (a) The major peak of activity was rerun on the same columns as shown in insert b, the peak fractions were taken for amino and terminal sequence analysis. (b) ¹²⁵l labelled protein samples of the fraction 22 (lane A,C) and 23 (lane B,D) were analyzed individually under unreduced (lane A,B) or reduced conditions (lane C,D).

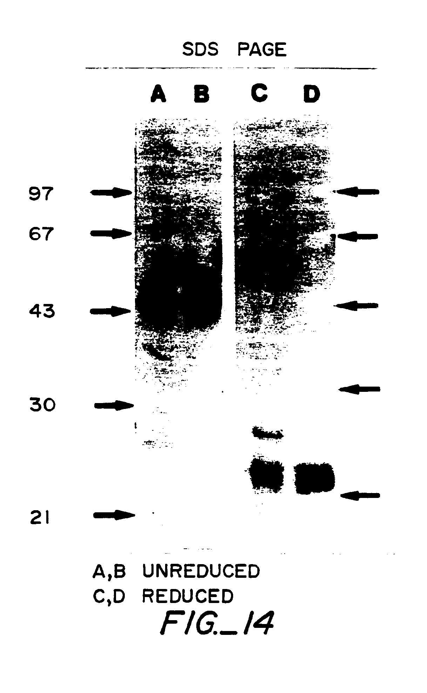

Fig. 14 - Electrophoresis was performed under the conditions described herein. Non-reduced conditions showed a component at about 43-45 kDa. The reduced conditions showed a component at about 23 kDa. After electrophoresis the gels were stained with Coomassie blue, destained, dried and subjected to autoradiography Migration of the samples was compared to that unreduced (left) or reduced (right) protein standards: 97,66,43,30,21 kDa.
Fig. 15 - Comparison of the ability of pituitary derived bFGF versus the AtT-20 cell derived growth factor to stimulate the growth of HUE cells (A), ACE cells (B), and BHK-21 cells (C).
Fig. 15A. Low density cultures of HUE cells(21) (5 x 10³ cells per 1 cm diameter gelatinized well) were exposed to HEPES (25 mM) buffered medium 199 supplemented with 100 µg/ml heparin, 10⁻⁸M selenium, 20% FSC (as described in (11) and increasing concentration of either pituitary derived bFGF (0) or AtT-20 cells derived growth factor (e). Heparin was added only once at the time of seeding while both bFGF and AtT-20 cells derived growth factor were added every other day. After 6 days in culture, triplicate wells were trypsinized and cell counted. The final density of cultures exposed to 20% FCS alone was 7.4 x 10⁴ cells per well. Standard deviation was less than 10% of the mean.
Fig. 15B. Low density cultures of ACE cells (5 x 10³ cells per 1 cm diameter well) were exposed to DMEM supplemented with 10% CS and increasing concentrations of either pituitary derived bFGF (e) or the AtT-20 cell derived growth factor (0) added every other day. After 5 days in cultures, triplicate wells were trysinized and cell counted. The final density of culture exposed to 10% CS alone was 1.3 x 10⁴ cells per well. Standard deviation was less than 10% of the mean.
Fig. 15C. Low density of BHK-21 cells(26) (2 x 10⁴ cells per 35 mm gelatinized tissue culture dishes) were exposed to 2 ml of DMEM-F12 (1 to 1 v/v) supplemented with 1.5 µg/ml gentamicin, 10 µg/ml transferring, 5 µg/ml insulin and increasing concentrations of either pituitary derived bFGF (e) or the AtT-20 cells derived growth factor (0). Insulin and transferrin were added only once, bFGF and AtT-20 cells derived growth facto were added every other day After 4 days in culture triplicate dishes were trypsinized and cell counted. The final density of culture exposed to transferrin insulin alone was 1.05 x 10⁴ cells/plate. Standard deviation was less than 10% of the mean.

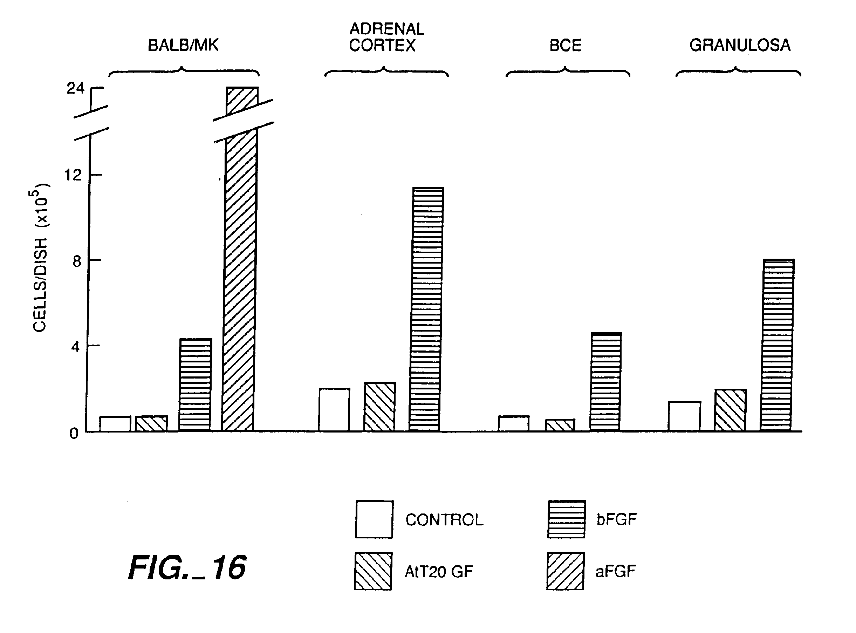

Fig. 16. BALB/MK cells(14) were seeded at a density of 3 x 10⁴ cells per 35 mm dishes in low Ca modified Eagle medium (15) supplemented with 10% FCS. 2 x 10⁴ BCE cells(16) granulosa cells(17), or adrenal cortex cells(18) per 35 mm dishes were seeded in their respective media (DMEM supplemented with 10% FCS, 5% CS, 10% CS for BCE and adrenal cortex cells and F-12 medium supplemented with 2.5% CS for granulosa cells). Pituitary derived bFGF (bFGF, 2 ng/mi) or AtT-20 cell derived growth factor (1.5 ng/ml) were added every other day. BALB/MK cells were also exposed to aFGF (10 ng/ml) since this mitogen is as potent as EGF in promoting their growth. After 6 days in cultures, cells were trypsinized and counted in a Coulter counter. Standard deviation was less than 10% of the mean.

An advantage of culturing the bovine cells to collect the growth factor is that the overall cell structure has good cohesive integrity That is to say, the cell layer usually remains intact for collecting the conditioning medium for many days, and even over a month or more of successive collection of the media samples. When the dome structure is present, it is possible to collect samples from above the dome and also from within or below the dome. Normally, the growth factor is in a higher concentration within the dome.

A disadvantage observed during the culturing of the bovine cells is that these cells produce a large amount of different proteins, which are secreted from the cell. Thus the purification steps need to be able to remove more of the undesired protein.

An advantage of the AtT-20 cell line is that it is commercially available.

An advantage of the culturing of the murine cells, AtT-20, is that these cells produce the novel growth factor without producing a large amount of other proteins which might interfere with the subsequent isolation steps.

A disadvantage of culturing the murine cells AtT-20 is that the structural integrity of the cell culture layer is not high. Thus during the culturing and collection, small portions of the cell layer will break away float in the conditioning media and then die. Often the culturing of the murine cells is only possible up to about 7 days or slightly longer.

The novel growth factor from bovine sources is often of sufficient purity after the mono S step and a Sephadex G-100 step that the RP HPCL-C4 purification is not necessary RP HPLC can be sued to determine the purity of the FsdGF.

### MURINE GROWTH FACTOR AMINO ACID SEQUENCE

The N-terminal sequence was determined for the growth factor protein derived from mouse AtT20 cells. Two N-terminal sequencing runs were carried out on the protein in an Applied Biosystems gas-phase protein sequencer. The second run, the sequencer was loaded with approximately 2.5 times as much of the protein as the first run.

As a result of comparing the data from the two sequencing runs, the following N-terminal sequence was determined:
X'-Pro-Thr-Thr-Glu-Gly-Glu-Gln-Lys-Ala His-Glu-Val-Val-Lys-Phe-Met-Asp-Val-Tyr-Gln-Arg-Ser-...

At amino acid position one (X')-three different amino acids were detected (Ala, Gly and Ser), with Ala being the most prominent. Since this amino acid residue was ambiguous, the residue is represented by a "X"'.

Comparison of the sequence from the mouse AtT20 cells with the N-terminal 23 amino acids determined for the bovine FSdGF protein demonstates that these two proteins are substantially homologous:
mouse
AtT20 prot.: X'-Pro-Thr-Thr-Glu-Gly-Glu-Gln-Lys-Ala His-Glu-Val-Val-Lys-Phe-Met-Asp-Val-Tyr-Gln-Arg-Ser-... bovine
FSdGF: Ala-Pro-Met-Ala-Glu-Gly-Gly-Gln-Lys-Pro-His-Glu-Val-Val-Lys-Phe-Met-Asp-Val-Tyr-Gln-Arg-Ser-...

The following Examples are provided to be illustrative and exemplary only. They are not to be construed as being limiting in anyway.

Reagents -- Tissue culture media and reagents were obtained from Gibco (Grand Island, N.Y). Acetonitrile and 2-propanol were purchased from Fisher Sci. (Fair Lawn, NJ). Heparin-sepharose (H-S) was obtained from Pharmacia (Piscataway, N.J.). Vydac HPLC columns were from The Separation Group (Hesperia, CA). Molecular weight markers for PAGE and protein determination kit were from Bio Rad Labs (Richmond, CA). Tissue culture plates were purchased from Costar, except for large scale Nunc plates (24.5 x 24.5 cm), which were Applied Sci. (San Francisco, CA). All other reagents were from Sigma Chemical Co. (St. Louis, MO) or Applied Biosystems (Foster City, CA).

### EXAMPLE A

Purpose of this Example was the determination of the molecular weight of the endothelial cell growth factor secreted in the medium by follicular cells.

Confluent cultures of follicular cells were incubated for three days in a serum-free medium consisting of low glucose Dulbecco's modified Eagle's medium supplemented with transferrin (10 µl/ml), insulin (5 µg/ml), 2 mM glutamine and antibiotics. The conditioned medium (CM) (150 ml) was then collected centrifuged (10000 xg, 15 min. 4°C) in order to remove cell debris, and then applied to a Heparin-Sepharose column which had been preequilibrated with 10 mM Tris/Cl, pH 7.0. The column was then sequentially eluted with 10 mM Tris/Cl, pH 7.0 containing 0.6, 1 and 3 M NaCI. The flow rate was 21 ml/h. Fractions of 700 pi were collected and aliquots were tested for bioactivity on adrenal cortex-derived microvascular endothelial cells. The majority of the bioactivity was eluted in the present of 0.6 M NaCI. This chromatographic behavior is different from that of aFGF or bFGF, which are known to elute, respectively, in the presence of 0.9-1.1 M NaCI and 1.8-2.2 M NaCI.

The most bioactive 0.6 NaCI fractions were pooled and further examined for determination of the molecular weight of growth factor activity A standard 12.5% polyacrilamide SDS slab gel was prepared. Ten percent glycerol and 2% SDS were added to the pooled fractions. Fifty per cent of the sample was treated with 2.5% 2-mercaptoethanol. The remainder 50% was not exposed to 2-mercaptoethanol or other reducing agents. The samples and prestained molecular weight markers were then incubated for 3 min at 37°C and electrophoresed overnight at a current of 10 mA. When the electrophoresis was completed, the gel was briefly rinsed in PBS and the distance of the molecular weight markers from the top of the gel was immediately meaured. Twenty-one half centimeter horizontal slices were then cut with a razor blade both from the lanes run under reducing and those run under non-reducing conditions. Slices were then washed twice with 1 ml of PBS and then shaken overnight at 4°C in individual tubes containing 500 gl of 0.2% gelatin in PBS for elution of the biological activity. The gel slices were then removed from the tubes, which were then centrifuged in order to remove particulate material. The supernatants were transferred to new tubes. Twenty microliter aliquots from each fraction were tested for biological activity on endothelial cells.

A single peak of bioactivity was observed in the group not exposed to 2-mercaptoethanol. The apparent molecular weight, as assessed by comparing the position of the molecular weight markers to that of the slices, was about 43,000. No bioactivity was recovered from slices exposed to 2-mercaptoethanol.

These results gave a good assessment of the molecular weight of the growth factor, which has been confirmed with the molecule purified to homogeneity, and also indicated that its activity is abolished by reducing agents.

### EXAMPLE 1

### CULTURE OF FOLLICULAR CELLS AND MEDIA COLLECTION

Primary cultures of bovine pituitary FC were established as previously described (20,41). In one embodiment in the culturing the 20% fetal bovine serum in reference 20 was reduced to 10%. Concentrations of 5 to 20% should be effective. Also no DNAase is used. All other components are the same. At confluency, cells were passaged into large scale tissue culture plates in the presence of low glucose Dulbecco's modified Eagle's medium (DMEM) supplemented with 10% fetal bovine serum, 2 mM glutamine and antibiotics. Shortly after reaching confluency the cultures were extensively washed with PBS in order to remove serum components. The cells were then incubated in a serum-free medium consisting of DMEM plus transferrin (10 µl/ml), insulin (5 wg/ml), selenium (10_{.}⁸ M), 2 mM glutamine and antibiotics. After three or four days, the medium was collected and replaced with fresh serum free medium. The collected medium was centrifuged (1000 g, 15 min. at 4°C) and stored at -70°C. The conditioned medium (CM) was then collected every three or four days for up to six weeks.

### EXAMPLE 2

### CONCENTRATION OF CONDITIONED MEDIUM

Four to six liter batches of conditioned medium (CM) were subjected to ammonium sulfate precipitation. Ammonium sulfate (500 g/L) was added under constant stirring, until the salt was completely in solution. After 8-12 hours in the cold room, the material was centrifuged (20,000 xg, 45 min at 4°C). The supernatant was discarded and the pellet was resuspended with 10 mM Tris/CI, pH 7.2, 50 mM NaCI and dialyzed at 4°C against the same buffer for 8-12 h. The final volume was 50-60 fold less than the original.

Alternatively, the CM is concentrated using ultrafiltration using an Amecon stir cell (2.5 liter unit) using a membrane having a molecular weight cut off of 10,000 daltons with similar results.

### EXAMPLE 3

### HEPARIN-SEPHAROSE AFFINITY CHROMATOGRAPHY

The concentrated CM was applied to a H-S column (14) (10 ml) preequilibrated with 10 mM Tris/Cl, pH 7.2, 50 mM NaCI. The column was then washed with the same buffer until the absorbance at 280 nm was negligible and then eluted stepwise with 10mM Tris/Cl pH 7.2 containing 0.15, 0.9 nd 3 M NaCI. The flow rate was 1.5 ml/min. Fractions of 1.5 ml were collected and aliquots, diluted with 0.2 % gelatin in PBS, were tested for mitogenic activity on endothelial cells.

### EXAMPLE 4

### REVERSE PHASE HPLC

(a) The most bioactive H-S fractions (0.9 M NaCI pool) were diluted fourfold with 0.1% trifluoroacetic acid (TFA) in water and applied to a Vydac C4 HPLC column (10 x 250 mm) preequilibrated in 0.1 TFA/20% acetonitrile. The column was eluted with a linear gradient of acetonitrile (20-45% in 115 min) at a flow rate of 2 ml/min. The absorbance was monitored at 21 nm. Fractions of 2 ml were diluted in 0.2% gelatin in PBS for assay on endothelial cells.
(b) The most bioactive fractions were pooled, diluted two fold in 0.1% TFA water and applied to a Vydac C4 HPLC column (4.6 x 250 mm) preequilibrated in 0.1% TFA/20% 2-propanol. The column was eluted with a linear gradient of 2-propanol (20-45% in 113 min). The flow rate was 0.6 ml/min. Aliquots of fractions were diluted for bioassays. The remainder of fractions were dried in a Speed-Va for SDS/PAGE (29) and structural analysis.

### EXAMPLE 5

### BIOASSAYS

Bovine adrenal cortex or brain-derived capillary endothelial cells, adult or fetal bovine aortic endothelial cells, human umbilical vein endothelial cells, bovine corneal endothelial cells, adrenal cortex cells, lens epithelial cells, BHK-21 fibroblasts and human keratynocytes were cultured and maintained as previously described (17,47,48,50,51,52,26,53). For bioassay, cells were seeded in the presence of their respective growth media at the density of 2 x 10⁴/35 mm dish or 1 x 10⁴/well in 12 multiwell plates. Fractions were added to cells in 5 µl/ml aliquots. After 4 or 5 days, cells were dissociated by exposure to trypsin and counted in a Coulter counter.

### EXAMPLE 6

### PROTEIN MICROSEQUENCING

Approximately 20 pmol of protein from the most bioactive fractions obtained from the second C4 step were applied directly to a gas phase protein sequenator Model 470A (Applied Biosystems). Edman degradation cycles were carried out and identification of amino acid derivatives was made by an on line HPLC column (54).

### EXPRESSION

Purification of growth factors is also described in U.S. Patents 4,708,948; 4,376,071; 4,350,687; 4,444,760; and 4,722,998.

The recombinant DNA production of growth factors is described in U.S. Patents 4,670,394; 4,721,672; 4,738,927; 4,783,412; and 4,801,542.

It is anticipated that the growth factors described herein will be able to be cloned and produced according to the methods described in the patents and references described herein.

It is understood that the growth factor described herein may be either the dimer (43,000 to 46,000 kDa) or the monomer (about 23 kDa).

While only a few embodiments of the invention have been shown and described herein, it will be come apparent to those skilled in the art that various modifications and changes can be made in the present invention to the novel endothelial cell growth factor, its methods of isolation, manufacture using recombinant DNA methods and its uses in therapy (wound healing).

## Claims

1. An endothelial cell growth factor which comprises a protein which is obtainable from folliculo stellate cells and which is in the form of a dimeric protein having a molecular weight of approximately 43-45kd under non-reducing conditions; or a fragment or variant thereof which has endothelial cell growth factor activity

2. An endothelial cell growth factor according to claim 1 which comprises an unglycosylated variant of a glycoprotein obtainable from folliculo stellate cells.

3. An endothelial cell growth factor according to claim 1 or claim 2 wherein the folliculo stellate cells are derived from a human or bovine source.

4. An endothelial cell growth factor according to claim 3 wherein the folliculo stellate cells are derived from a human.

5. An endothelial cell growth factor according to any one of claims 1 to 3 which comprises at its N-terminus the amino acid sequence Ala-Pro-Met-Ala-Glu-Gly-Gly-Gln-Lys-Pro-His-Glu.

6. An endothelial cell growth factor according to claim 5 which comprises at its N-terminus the amino acid sequence Ala-Pro-Met-Ala-Glu-Gly-Gly-Gln-Lys-Pro-His-Glu-Val-Val-Lys-Phe-Met-Asp-Val-Tyr-Gln.

7. An endothelial cell growth factor according to claim 6 having an N-terminus amino acid sequence of: Ala-Pro-Met-Ala-Glu-Gly-Gly-Gln-Lys-Pro-His-Glu-Val-Val-Lys-Phe-Met-Asp-Val-Tyr-Gln-(Arg)-Ser-Phe-X-Arg-Pro-Ile-Glu-Thr-Leu-(Val)-X-lie-X-(Gin)-Glu-Tyr-(Pro)- wherein the amino acids in parenthesis are uncertain and the-X indicates an amino acid of unknown identity.

8. An endothelial cell growth factor according to any one of the previous claims which has a molecular weight of approximately 43Kd.

9. An endothelial cell growth factor according to claim 8 which, under reducing conditions, forms two substantially homologous units, each unit having a molecular weight of about 23,000 daltons.

10. An endothelial cell growth factor according to any one of the preceding claims which further comprises an internal amino acid sequence, obtainable upon tryptic digestion, which sequence comprises Ser-Phe-Cys-Arg-Pro-lIe-Glu-Thr-Leu-Val-Ssp-Ile-Phe-Gln-Glu-Tyr-Pro-Asp-Glu-Ile; and/or
Ser-Phe-Cys-Arg-Pro-Ile-Glu-Thr-Leu-Val-Ssp-Ile-Phe-Gln-Glu-Tyr-Pro-Asp/Ile Glu.

11. An endothelial cell growth factor according to claim 1 which is obtainable from a murine source and/or which comprises at its N-terminus the amino acid sequence X'-Pro-Thr-Thr-Glu-Gly-Glu-Gln-Lys-Ala-His-Glu-Val-Val-Lys-Phe-Met-Arp-Val-Tyr-Gln-Arg-Ser- when X' is either Ala, Gly or Ser.

12. A method of promoting the proliferation of endothelial cells in-vitro which comprises applying to such cells an endothelial cell growth factor according to any one of claims 1 to 11.

13. A method according to claim 12, wherein the endothelial cells are grown in cell culture.

14. A method of obtaining a substantially pure endothelial cell growth factor according to claim 3, which method comprises:
(a) providing a biological sample from a bovine or human subject containing levels of endothelial cell growth factor.
(b) extracting a solubilized portion of the sample;
(c) partially purifying the endothelial cell growth factor from the extract using an aqueous salt-precipitation step;
(d) fractionating the purified extract using affinity chromatograph employing heparin moieties linked to an insoluble support as the stationary phase and employing a step gradient mobile phase of increasing salt concentration;
(e) fractionating the extract using gel exclusion chromatography; and optionally
(f) purifying the extract using reverse-phase high pressure liquid chromatography.

15. A method according to claim 14 wherein the step (c) the aqueous salt precipitation uses ammonium sulphate.

16. A method according to claim 14 or claim 15 wherein:
in fractionating step (d) the heparin is attached to sepharose (Registered Trade Mark); and
in the purifying step (f) the reverse phase high performance liquid chromatography is conducted using an acetonitrile gradient.

17. A method of obtaining a concentrated endothelial cell growth factor according to claim 1, said method comprising:
(a) obtaining a sample of conditioned medium of a folliculo stellate cell culture containing levels of said growth factor;
(b) fractionating the sample of step (a) using heparin moieties linked to an insoluble support as the stationary phase and using a salt gradient mobile phase of increasing salt concentration; and
(c) purifying the bioactive fraction of step (b) using gel electrophoresis.

18. A method according to claim 17 wherein in the fractionating step the growth factor elutes at a sodium chloride concentration of between about 0.6 and 1.0 Molar.

19. A method according to claim 18 wherein in the purifying step the gel is a polyacrylamide.

20. A method according to any one of claims 17 to 19 wherein the culture from which said conditioned medium is obtained from is a murine source.

21. A method according to claim 20 wherein the culture is a culture of AtT20 cells.

22. A pharmaceutical composition comprising the endothelial cell growth factor of any one of claims 1 to 11 and a pharmaceutically acceptable carrier vehicle.

23. A pharmaceutical composition according to claim 22 wherein the carrier vehicle is a parenteral carrier vehicle.

24. A composition according to claim 22 or claim 23 which is adapted for wound healing in a human being by presenting the growth factor at a concentration of between about 10 picogram/milliliter and about 500 picogram/milliliter.

25. A method according to claim 14 wherein after step (e) and before step (f) there is provided a further step of fractionating the sample using liquid chromatography

26. A method according to claim 25 wherein the liquid chromatography is cation exchange chromatography

27. A method according to any one of claims 25 to 26 wherein in step (f) the reverse phase high pressure liquid chromatography is performed using an aqueous acetonitrile gradient.

28. A method according to any one of claims 25 to 27 which further includes:
(g) purifying the concentrated product of step (f) using reverse phase high performance liquid chromatography with an aqueous isopropanol gradient.

29. A nucleic acid which encodes an endothelial cell growth factor according to any one of claims 1 to 11.

30. A replicable expression vector which includes a deoxyribonucleic acid according to claim 29.

31. A replicable expression vector according to claim 30 which comprises a bacteriophage.

32. A replicable expression vector according to claim 30 which is a plasmid.

33. A recombinant host cell transformed using an expression vector according to any one of claims 30-32.

34. A process for producing an endothelial cell growth factor according to any one of claims 1 to 11, which method comprises maintaining a recombinant host cell according to claim 33, under conditions permitting expression of said endothelial cell growth factor.

35. The process according to claim 34 including the further step of recovering said endothelial cell growth factor.

36. An endothelial cell growth factor obtainable by the method of claim 14.

37. A method of obtaining a substantially pure endothelial cell growth factor according to claim 1 which method comprises:
(a) obtaining a biological sample containing levels of said endothelial cell growth factor using the conditioned medium of a folliculo stellate cell culture;
(b) extracting a solubilised portion of the sample;
(c) partially purifying the endothelial cell growth factor from the extract using an aqueous salt-precipitation step;
(d) fractionating the purified extract using affinity chromatography employing heparin moieties linked to an insoluble support as the stationary phase and employing a step gradient mobile phase of increasing salt concentration;
(e) purifying the extract using reverse-phase high pressure liquid chromatography; and
(f) further purifying the extract using a second reverse-phase high pressure liquid chromatography.

38. A method according to claim 37 wherein the step (e) utilises acetronitrile gradient.

39. A method according to claim 37 or claim 38 wherein in step (f), propanol is used.

40. A method according to any one of claims 37 to 39 wherein the aqueous salt precipitation step (c) comprises an ammonium salt precipitation step.

41. An endothelial cell growth factor according to any one of claims 1 to 11 for use in the treatment of wounds.

42. The use of an endothelial cell growth factor according to any one of claims 1 to 11 for use in the preparation of a medicament for the treatment of wounds.

## Patentansprüche

1. Endothelzellenwachstumsfaktor, umfassend ein Protein, das aus sternförmigen Follikelzellen erhalten werden kann und das in Form eines dimeren Proteins mit einem Molekulargewicht von etwa 43-45 kD unter nichtreduzierenden Bedingungen vorliegt; oder ein Fragment oder eine Variante davon, die Endothelzellenwachstumsfaktor-Aktivität aufweist.

2. Endothelzellenwachstumsfaktor nach Anspruch 1, umfassend eine unglykosylierte Variante eines Glykoproteins, das aus sternförmigen Follikelzellen erhalten werden kann.

3. Endothelzellenwachstumsfaktor nach Anspruch 1 oder 2, worin die sternförmigen Follikelzellen aus einem Menschen oder einem Rind stammen.

4. Endothelzellenwachstumsfaktor nach Anspruch 3, worin die sternförmigen Follikelzellen aus einem Menschen stammen.

5. Endothelzellenwachstumsfaktor nach einem der Ansprüche 1 bis 3, der an seinem N-Terminus die Aminosäuresequenz ¹A1a-Pro-Met-Ala-Glu-Gly-Gly-Gln-Lys-Pro-His-Glu umfaßt.

6. Endothelzellenwachstumsfaktor nach Anspruch 5, der an seinem N-Terminus die Aminosäuresequenz Ala-Pro-Met-Ala-Glu-Gly-Gly-Gln-Lys-Pro-His-Glu-Val-Val-Lys-Phe-Met-Asp-Val-Tyr-Gln umfaßt.

7. Endothelzellenwachstumsfaktor nach Anspruch 6 mit einer N-terminalen Aminosäuresequenz von Ala-Pro-Met-Ala-Glu-Gly-Gly-Gln-Lys-Pro-His-Glu-Val-Val-Lys-Phe-Met-Asp-Val-Tyr-Gln-(Arg)-Ser-Phe-X-Arg-Pro-Ile-Glu-Thr-Leu-(Val)-X-lIe-X-(Gln)-Glu- Tyr-(Pro)-, worin die Aminosäuren in Klammern unsicher sind und das -X eine Aminosäure unbekannter Identität anzeigt.

8. Endothelzellenwachstumsfaktor nach einem der vorhergehenden Ansprüche, der ein Molekulargewicht von etwa 43 kD aufweist.

9. Endothelzellenwachstumsfaktor nach Anspruch 8, der unter reduzierenden Bedingungen zwei im wesentlichen homologe Einheiten bildet, wobei jede Einheit ein Molekulargewicht von etwa 23.000 Dalton aufweist.

10. Endothelzellenwachstumsfaktor nach einem der vorhergehenden Ansprüche, weiters umfassend eine interne Aminosäuresequenz, die nach tryptischer Spaltung erhalten werden kann, welche Sequenz folgendes umfaßt: Ser-Phe-Gys-Arg-Pro-Ile-Glu-Thr-Leu-Val-Asp-lle-Phe-Gln-Glu-Tyr-Pro-Asp-Glu-lle; und/oder Ser-Phe-Cys-Arg-Pro-Ile-Glu-Thr-Leu-Val-Asp-Ile-Phe-Gln-Glu-Tyr-Pro-Asp/Ile Glu.

11. Endothelzellenwachstumsfaktor nach Anspruch 1, der aus einer Maus erhalten werden kann und/oder an seinem N-Terminus die Aminosäure X'-Pro-Thr-Thr-Glu-Gly-Glu-Gln-Lys-Ala-His-Glu-Val-Val-Lys-Phe-Met-Asp-Val-Tyr-Gln-Arg-Ser- aufweist, worin X' entweder Ala, Gly oder Ser ist.

12. Verfahren zur Förderung der Proliferation von Endothelzellen in vitro, umfassend das Versehen solcher Zellen mit einem Endothelzellenwachstumsfaktor nach einem der Ansprüche 1 bis 11.

13. Verfahren nach Anspruch 12, worin die Endothelzellen in Zellkultur gezüchtet werden.

14. Verfahren zum Erhalten eines im wesentlichen reinen Endothelzellenwachstumsfaktors nach Anspruch 3, welches Verfahren folgendes umfaßt:
(a) Bereitstellen einer biologischen Probe aus einem Rind oder einem menschlichen Probanden, die Mengen an Endothelzellenwachstumsfaktor enthält;
(b) Extrahieren eines solubilisierten Teils der Probe;
(c) teilweises Reinigen des Endothelzellenwachstumsfaktors aus dem Extrakt unter Anwendung eines wäßrigen Salz-Ausfällungsschritts;
(d) Fraktionieren des gereinigten Extrakts mittels einer Affinitätschromatografie unter Verwendung von Heparingruppen, die mit einem unlöslichen Träger als stationäre Phase verbunden sind, und mittels eines Stufengradienten der mobilen Phase mit steigender Salzkonzentration;
(e) Fraktionieren des Extrakts mittels Gelausschlußchromatografie; und gegebenenfalls
(f) Reinigen des Extrakts durch Umkehrphasen-Hochdruck-Flüssigkeitschromatografie.

15. Verfahren nach Anspruch 14, worin in Schritt (c) der wäßrigen Salzausfällung Ammoniumsulfat verwendet wird.

16. Verfahren nach Anspruch 14 oder 15, worin:
im Fraktionierungsschritt (d) das Heparin an Sepharose™ befestigt ist; und
im Reinigungsschritt (f) die Umkehrphasen-Hochleistungs-Flüssigkeitschromatografie mittels eines Acetonitrilgradienten durchgeführt wird.

17. Verfahren zum Erhalten eines konzentrierten Endothelzellenwachstumsfaktors nach Anspruch 1, welches Verfahren folgendes umfaßt:
(a) Erhalten einer Probe von konditioniertem Medium aus einer Kultur von sternförmigen Follikelzellen, die Mengen des Wachstumsfaktors enthält;
(b) Fraktionieren der Probe von Schritt (a) mittels Heparingruppen, die mit einem unlöslichen Träger als stationäre Phase verbunden sind, und Verwendung eines Salzgradienten der mobilen Phase mit steigender Salzkonzentration; und
(c) Reinigen der bioaktiven Fraktion von Schritt (b) mittels Gelelektrophorese.

18. Verfahren nach Anspruch 17, worin im Fraktionierungsschritt der Wachstumsfaktor bei einer Natriumchloridkonzentration von zwischen etwa 0,6 und 1,0 Mol/l eluiert.

19. Verfahren nach Anspruch 18, worin das Gel im Reinigungsschritt ein Polyacrylamid ist.

20. Verfahren nach einem der Ansprüche 17 bis 19, worin die Kultur, aus der das konditionierte Medium erhalten wird, aus einer Maus stammt.

21. Verfahren nach Anspruch 20, worin die Kultur eine Kultur von AtT20-Zellen ist.

22. Pharmazeutische Zusammensetzung, umfassend den Endothelzellwachstumsfaktor nach einem der Ansprüche 1 bis 11 und ein pharmazeutisch annehmbares Trägervehikel.

23. Pharmazeutische Zusammensetzung nach Anspruch 22, worin das Trägervehikel ein parenterales Trägervehikel ist.

24. Zusammensetzung nach Anspruch 22 oder 23, die zur Wundheilung in einem Menschen geeignet ist, indem der Wachstumsfaktor bei einer Konzentration von zwischen etwa 10 pg/ml und etwa 500 pg/ml vorliegt.

25. Verfahren nach Anspruch 14, worin nach Schritt (e) und vor Schritt (f) ein weiterer Schritt des Fraktionierens der Probe mittels Flüssigkeitschromatografie vorgesehen ist.

26. Verfahren nach Anspruch 25, worin die Flüssigkeitschromatografie eine Kationenaustauschchromatografie ist.

27. Verfahren nach einem der Ansprüche 25 bis 26, worin in Schritt (f) die Umkehrphasen-Hochleistungs-Flüssigkeitschromatografie unter Verwendung eines wäßrigen Actonitrilgradienten durchgeführt wird.

28. Verfahren nach einem der Ansprüche 25 bis 27, das weiters umfaßt:
(g) Reinigen des konzentrierten Produkts von Schritt (f) mittels Umkehrphasen-Hochleistungs-Flüssigkeitschromatografie mit einem wäßrigen Isopropanolgradienten.

29. Nukleinsäure, die für einen Endothelzellenwachstumsfaktor nach einem der Ansprüche 1 bis 11 kodiert.

30. Replizierbarer Expressionsvektor, der eine Desoxyribonukleinsaure nach Anspruch 29 umfaßt.

31. Replizierbarer Expressionsvektor nach Anspruch 30, der einen Bakteriophagen umfaßt.

32. Replizierbarer Expressionsvektor nach Anspruch 30, der ein Plasmid ist.

33. Rekombinante Wirtszelle, die unter Verwendung eines Expressionsvektors nach einem der Ansprüche 30-32 transformiert ist.

34. Verfahren zur Herstellung eines Endothelzellenwachstumsfaktors nach einem der Ansprüche 1 bis 11, welches Verfahren das Halten einer rekombinanten Wirtszelle nach Anspruch 33 unter Bedingungen, die eine Expression des Endothelzellenwachstumsfaktors ermöglichen, umfaßt.

35. Verfahren nach Anspruch 34, umfassend den weiteren Schritt des Gewinnens des Endothelzellenwachstumsfaktors.

36. Endothelzellenwachstumsfaktor, der durch das Verfahren nach Anspruch 14 erhalten werden kann.

37. Verfahren zum Erhalten eines im wesentlichen reinen Endothelzellenwachstumsfaktors nach Anspruch 1, welches Verfahren folgendes umfaßt:
(a) Erhalten einer biologischen Probe, die Mengen des Endothelzellenwachstumsfaktors enthält, unter Verwendung des konditionierten Mediums aus einer Kultur von sternförmigen Follikelzellen;
(b) Extrahieren eines solubilisierten Teils der Probe;
(c) teilweises Reinigen des Endothelzellenwachstumsfaktors aus dem Extrakt unter Anwendung eines wäßrigem Salz-Ausfällungsschritts;
(d) Fraktionieren des gereinigten Extrakts mittels Affinitätschromatografie unter Verwendung von Heparingruppen, die mit einem unlöslichen Träger als stationäre Phase verbunden sind, und unter Verwendung eines Stufengradienten der mobilen Phase mit steigender Salzkonzentration;
(e) Reinigen des Extrakts mittels Umkehrphasen-Hochleistungs-Flüssigkeitschromatografie; und
(f) weiteres Reinigen des Extrakts mittels einer zweiten Umkehrphasen-Hochleistungs-Flüssigkeitschromatografie.

38. Verfahren nach Anspruch 37, worin in Schritt (e) ein Acetonitrilgradient verwendet wird.

39. Verfahren nach Anspruch 37 oder 38, worin in Schritt (f) Propanol verwendet wird.

40. Verfahren nach einem der Ansprüche 37 bis 39, worin der wäßrige Salz-Ausfällungsschritt (c) einen Ammoniumsalz-Ausfällungsschritt umfaßt.

41. Endothelzellenwachstumsfaktor nach einem der Ansprüche 1 bis 11 zur Verwendung bei der Wundbehandlung.

42. Verwendung eines Endothelzellenwachstumsfaktors nach einem der Ansprüche 1 bis 11 zur Verwendung bei der Herstellung eines Medikaments zur Wundbehandlung.

## Revendications

1. Facteur de croissance des cellules endothéliales qui comprend une protéine qui peut être obtenue à partir des cellules stellaires des follicules et qui est sous la forme d'une protéine dimérique ayant un poids moléculaire d'approximativement 43 à 45 kd dans des conditions non réductrices; ou un fragment ou variant de celui-ci qui a une activité de facteur de croissance des cellules endothéliales.

2. Facteur de croissance des cellules endothéliales selon la revendication 1 qui comprend un variant non glycosylé d'une glycoprotéine qui peut être obtenue à partir des cellules stellaires des follicules.

3. Facteur de croissance des cellules endothéliales selon la revendication 1 ou la revendication 2 dans lequel les cellules stellaires des follicules sont dérivées d'une source humaine bovine.

4. Facteur de croissance des cellules endothéliales selon la revendication 3 dans lequel les cellules stellaires des follicules sont dérivées d'un humain.

5. Facteur de croissance des cellules endothéliales selon l'une quelconque des revendications 1 à 3 qui comprend à son terminus N la séquence d'acides aminés Ala-Pro-Met-Ala-Glu-Gly-Gly-Gln-Lys-Pro-His-Glu.

6. Facteur de croissance de cellules endothéliales selon la revendication 5 qui comprend à son terminus N la séquence d'acides aminés Ala-Pro-Met-Ala-Glu-Gly-Gly-Gln-Lys-Pro-His-Glu-Val-Val-Lys-Phe-Met-Asp-Val-Tyr-Gln.

7. Facteur de croissance des cellules endothéliales selon la revendication 6 ayant une séquence d'acides aminés au terminus N : Ala-Pro-Met-Ala-Glu-Gly-Gly-Gln-Lys-Pro-His-Glu-Val-Val-Lys-Phe-Met-Asp-Val-Tyr-Gln-(Arg)-Ser-Phe-X-Arg-Pro-Ile-Glu-Thr-Leu-(Val)-X-Ile-X-(Gln)-Glu-Tyr-(Pro) où les acides aminés entre parenthèses sont incertains et où le -X indique un acide aminé d'identité inconnue.

8. Facteur de croissance des cellules endothéliales selon l'une quelconque des revendications précédentes qui a un poids moléculaire d'approximativement 43 Kd.

9. Facteur de croissance des cellules endothéliales selon la revendication 8 qui, dans des conditions réductrices, forme deux unités substantiellement homologues, chaque unité ayant un poids moléculaire d'environ 23 000 daltons.

10. Facteur de croissance des cellules endothéliales selon l'une quelconque des revendications précédentes qui comprend de plus une séquence interne d'acides aminés, qui peut être obtenue par digestion tryptique, laquelle séquence comprend Ser-Phe-Cys-Arg-Pro-Ile-Glu-Thr-Leu-Val-Ssp-Ile-Phe-Gln-Glu-Tyr-Pro-Asp-Glu-11e; et/ou Ser-Phe-Cys-Arg-Pro-Ile-Glu-Thr-Leu-Val-Ssp-Ile-Phe-Gln-Glu-Tyr-Pro-Asp/Ile Glu.

11. Facteur de croissance des cellules endothéliales selon la revendication 1 qui peut être obtenu à partir d'une source murine et/ou qui comprend à son terminus-N la séquence d'acides aminés X'-Pro-Thr-Thr-Glu-Gly-Glu-Gln-Lys-Ala-His-Glu-Val-Val-Lys-Phe-Met-Arp-Val-Tyr-Gln-Arg-Ser- où X' est soit Ala, soit Gly soit Ser.

12. Méthode pour promouvoir la prolifération des cellules endothéliales in-vitro qui comprend l'application à de telles cellules d'un facteur de croissance de cellules endothéliales selon l'une quelconque des revendications 1 à 11.

13. Méthode selon la revendication 12, dans laquelle les cellules endothéliales sont mises à croître dans une culture de cellules.

14. Méthode d'obtention d'un facteur de croissance des cellules endothéliales substantiellement pur selon la revendication 3, laquelle méthode comprend :
(a) la fourniture d'un échantillon biologique provenant d'un sujet bovin ou humain contenant des niveaux d'un facteur de croissance des cellules endothéliales.
(b) l'extraction d'une portion solubilisée de l'échantillon;
(c) la purification partielle du facteur de croissance des cellules endothéliales provenant de l'extrait en utilisant une étape de précipitation par un sel aqueux;
(d) le fractionnement de l'extrait purifié en utilisant une chromatographie d'affinité en employant des moitiés héparine pontées à un support insoluble en tant que phase stationnaire et en employant une phase mobile à gradient de sel à une concentration de sel croissante;
(e) le fractionnement de l'extrait en utilisant une chromatographie d'exclusion sur gel; et optionnellement
(f) la purification de l'extrait en utilisant une chromatographie liquide à haute pression à phase inverse.

15. Méthode selon la revendication 14 dans laquelle l'étape (c) de précipitation par un sel aqueux utilise du sulfate d'ammonium.

16. Méthode selon la revendication 14 ou la revendication 15 dans laquelle :
dans l'étape de fractionnement (d) l'héparine est liée à de la sépharose (Marque Enregistrée); et
dans l'étape de purification (f) la chromatographie liquide à haute performance à phase inverse est menée en utilisant un gradient d'acétonitrile.

17. Méthode d'obtention d'un facteur de croissance des cellules endothéliales concentré selon la revendication 1, ladite méthode comprenant :
(a) l'obtention d'un échantillon d'un milieu conditionné d'une culture de cellules stellaires de follicules contenant des niveaux dudit facteur de croissance;
(b) le fractionnement de l'échantillon de l'étape (a) en utilisant des moitiés héparine pontées à un support insoluble en tant que phase stationnaire et en utilisant une phase mobile de gradient de sel d'une concentration en sel croissante; et
(c) la purification de la fraction bioactive de l'étape (b) en utilisant une électrophorèse sur gel.

18. Méthode selon la revendication 17 dans laquelle dans l'étape de fractionnement le facteur de croissance élue à une concentration de chlorure de sodium entre environ 0,6 et 1,0 Molaire.

19. Méthode selon la revendication 18 dans laquelle dans l'étape de purification le gel est un polyacrylamide.

20. Méthode selon l'une quelconque des revendications 17 à 19 dans laquelle la culture à partir de laquelle ledit milieu conditionné est obtenu est une source murine.

21. Méthode selon la revendication 20 dans laquelle la culture est une culture de cellules AtT20.

22. Composition pharmaceutique comprenant le facteur de croissance des cellules endothéliales selon l'une quelconque des revendications 1 à 11 et un véhicule porteur pharmaceutiquement acceptable.

23. Composition pharmaceutique selon la revendication 22 dans laquelle le véhicule porteur est un véhicule porteur parentéral.

24. Composition selon la revendication 22 ou la revendication 23 qui est adaptée pour guérir une blessure chez un être humain par présentation du facteur de croissance à une concentration d'entre environ 10 picogrammes/millilitre et environ 500 picogrammes/millilitre.

25. Méthode selon la revendication 14 dans laquelle après l'étape (e) et avant l'étape (f) il est prévu une étape supplémentaire de fractionnement de l'échantillon en utilisant une chromatographie liquide.

26. Méthode selon la revendication 25 dans laquelle la chromatographie liquide est une chromatographie d'échange de cations.

27. Méthode selon l'une quelconque des revendications 25 à 26 dans laquelle dans l'étape (f) la chromatographie liquide à haute pression à phase inverse est effectuée en utilisant un gradient d'acétonitrile aqueux.

28. Méthode selon l'une quelconque des revendications 25 à 27 qui inclut de plus :
(g) la purification du produit concentré de l'étape (f) en utilisant une chromatographie liquide à haute performance à phase inverse avec un gradient d'isopropanol aqueux.

29. Acide nucléique qui encode un facteur de croissance des cellules endothéliales selon l'une quelconque des revendications 1 à 11.

30. Vecteur d'expression réplicable qui inclut un acide désoxyribonucléique selon la revendication 29.

31. Vecteur d'expression réplicable selon la revendication 30 qui comprend un bactériophage.

32. Vecteur d'expression réplicable selon la revendication 30 qui est un plasmide.

33. Cellule hôte recombinante transformée en utilisant un vecteur d'expression selon l'une quelconque des revendications 30 à 32.

34. Procédé pour produire un facteur de croissance des cellules endothéliales selon l'une quelconque des revendications 1 à 11, laquelle méthode comprend le maintien d'une cellule hôte recombinante selon la revendication 33, dans des conditions permettant l'expression dudit facteur de croissance des cellules endothéliales.

35. Procédé selon la revendication 34 incluant l'étape supplémentaire de récupération dudit facteur de croissance des cellules endothéliales.

36. Facteur de croissance des cellules endothéliales qui peut être obtenu par la méthode selon la revendication 14.

37. Méthode d'obtention d'un facteur de croissance des cellules endothéliales substantiellement pur selon la revendication 1 laquelle méthode comprend :
(a) obtention d'un échantillon biologique contenant des niveaux dudit facteur de croissance des cellules endothéliales en utilisant le milieu conditionné d'une culture de cellules stellaires de follicules;
(b) l'extraction d'une portion solubilisée de l'échantillon;
(c) la purification partielle du facteur de croissance des cellules endothéliales provenant de l'extrait en utilisant une étape de précipitation par un sel aqueux;
(d) le fractionnement de l'extrait purifié en utilisant une chromatographie d'affinité en employant des moitiés héparines pontées à un support insoluble en tant que phase stationnaire et en employant une phase mobile à gradient par étape d'une concentration de sel croissante;
(e) la purification de l'extrait en utilisant une chromatographie liquide à haute pression à phase inverse; et
(f) la purification supplémentaire de l'extrait en utilisant une seconde chromatographie liquide à haute pression à phase inverse.

38. Méthode selon la revendication 37 dans laquelle l'étape (e) utilise un gradient d'acétonitrile.

39. Méthode selon la revendication 37 ou la revendication 38 dans laquelle dans l'étape (f), le propanol est utilisé.

40. Méthode selon l'une quelconque des revendications 37 à 39 dans laquelle l'étape (c) de précipitation par un sel aqueux comprend une étape de précipitation par un sel d'ammonium.

41. Facteur de croissance de cellules endothéliales selon l'une quelconque des revendications 1 à 11 pour utilisation dans la préparation d'un médicament pour le traitement des blessures.

42. Utilisation d'un facteur de croissance selon l'une quelconque des revendications 1 à 11 pour l'utilisation dans la préparation d'un médicament pour le traitement de blessures.
